# EUROPEAN PATENT APPLICATION

(11) **EP 4 296 669 A1**
(43) Date of publication of application: **27.12.2023**
(21) Application number: 22179990.1
(22) Date of filing: 20.06.2022
(51) Int. Cl.: G01N 33/50, C12P 21/02, G01N 33/68

(54) **HIGH-THROUGHPUT SCREENING FOR LIGANDS OF TRANSMEMBRANE PROTEINS**

(71) Applicant: LenioBio GmbH, 40231 Düsseldorf (DE)
(72) Inventor: Das Gupta, Mainak, Ulm (DE)
(74) Representative: Heide, Anna Katharina

(57) **Abstract**

The present invention relates to a high-throughput screening for at least one ligand of at least one transmembrane protein (TP) of interest that is embedded in a lipid bilayer of at least one endogenous microsome derived from plant-based endoplasmatic reticulum (ER), comprising the steps providing at least one endogenous microsome comprising at least one lipid bilayer embedded TP or at least one endogenous microsomal fragment comprising at least one lipid bilayer embedded TP, providing at least one analyte of interest, contacting of the at least one TP with the at least one analyte and detection of interaction between the at least one analyte and the at least one TP. The screening is suitable for high multiplex grades of TPs and analytes and for a fast and reproducible identification of ligands as potential drug candidates. Additionally, the invention provides all essential products, consumables and kits for use in the high-throughput screening.

## Description

The present invention relates to a high-throughput screening (HTS) for at least one ligand of at least one transmembrane protein (TP) of interest that is embedded in a lipid bilayer of at least one endogenous microsome derived from plant-based endoplasmatic reticulum (ER), comprising the steps of providing at least one endogenous microsome comprising at least one lipid bilayer embedded TP or at least one endogenous microsomal fragment comprising at least one lipid bilayer embedded TP, providing at least one analyte of interest, contacting of the at least one TP with the at least one analyte and detection of interaction between the at least one analyte and the at least one TP. The screening is suitable for high multiplex grades of TPs and analytes and for a fast and reproducible identification of ligands as potential drug candidates. Additionally, the inventions provide all essential products, consumables and kits for use in the high-throughput screening. Therefore the present invention relates to the technical field of biochemical and biotechnological drug candidate screening for TPs which are associated with signalling responsible for or involved in a disease.

Presently, as a proof-of-concept, a cell-free (CF) system based on lysates derived from the BY-2 cells of Nicotiana tabacum that harbours endogenous microsomes called ALiCE^{®}, was used for TP production. Three well-characterized TPs: hEGF, PVR, TIGIT and ACE2, were used for expression in ALiCE^{®}, using the pALiCE02 vector (LenioBio GmbH). Furthermore, C-terminally tagged TP were used for the proof-of-concept of the HTS according to the invention and microsomes were captured. Once TPs were captured, interaction (binding) assays of TPs and their respective ligand were carried out. ALiCE^{®} proved to be a successful CF system for the production of the type I transmembrane proteins PVR, TIGIT and ACE2. The expression of these proteins contributed to the design of assays to study protein orientation and structure. Additionally, PVR and ACE2 where correctly folded and binding assays confirmed that ACE2 could bind to RBD. TP expression in ALiCE^{®} and microsome capturing assays proves the HTS of the present invention for high throughput membrane protein drug target screening platforms.

Drug discovery is an arduous, expensive and time-consuming process. The average time to develop a new drug is 10-15 years and it can take up to 800 million euros. The SARS-CoV-2 pandemic has shown that this process needs to speed up for a quick response against the threat of new pandemics in the upcoming years. Target-based drug discovery is the most common strategy for the development of new drugs. Traditionally, the most followed paradigm constitutes one drug to one target, but the need for a platform that allows screening of multiple drugs against one target has become essential. Ligand screening for drug discovery is crucial for the development of new pharmaceuticals. Currently, there are two major approaches for drug discovery, which are target-based screening or phenotypic screening. Phenotypic screening is based on trying to identify molecules that can alter the cell's phenotype towards a desired outcome, while target-based screening is based on the use of recombinant technology to find a target that plays a role in a disease. Since the 1990s, High Throughput Screening (HTS), a target focused screening, has increasingly been used for the discovery of new drugs by most pharmaceutical companies. For instance, HTS is frequently used for research on possible drugs against neurodegenerative diseases. HTS is based on the screening of multiple molecules/compounds or "hits" against one known target. The increasing use of HTS has replaced slow paced typically used "trial and error" techniques to establish a quicker method. The throughput of the prior art screening process however, is limited, showing an existing need to develop next generation drug-screening platforms at higher throughput to increase the speed of drug discovery. The main characteristic of HTS is that the target for drug discovery is known and tests with multiple molecules that act on the target can be analysed as possible drugs. A drug target may be a small molecule or any kind of protein that plays a role in a particular disease process and could be addressed by a drug to treat this disease. For this reason, the pharmaceutical industry depends on the selective binding of the "hit" to the target or specific interaction causing a modulation of the target.

However, HTS presents some limitations that prevent reaching its full potential, in particular HTS for drug candidates of transmembrane proteins (TPs), which constitute the majority of drug targets but which are underrepresented compared to cytosolic proteins. Currently, more than 60% of the existing drug targets are transmembrane proteins (TPs). TPs are involved in essential physiological mechanisms ensuring structural and functional integrity of cells. For many processes such as cell recognition, immune response, signal transduction, and transport of molecules TPs are the key components. The use of TPs constitutes a problem for drug development since expression, post-translational modification (PTM), protein folding and stabilization during HTS is difficult.

TPs make up 30% of the total proteins encoded by humans and yet they are poorly characterized. TPs are difficult to produce in living eukaryotic cells. One of the main problems in research is that the overexpression of TPs has strongly deleterious effects on eukaryotic cell health, thereby hampering the production of correctly folded and physiologically functional TPs. The limitations in the production of this type of protein is responsible for the delay on functional and structural characterization of TPs. Additionally, prokaryotic cells are not suited for eukaryotic TP production and the expression of these proteins cause the formation of protein aggregates, which need to be solubilized after expression (Sachse et al., 2013). Nevertheless, there are other protein expression platforms that allow the production of many types of proteins in a simple way (Khambhati et al., 2019) without depending on the structural integrity of a cell. These platforms are based on cell-free protein synthesis (CFPS).

Cell-free protein synthesis (CPSF) systems are based on crude lysates that contain the necessary machinery for translation, protein folding, and energy metabolism (Buntru et al., 2014). CFPS is a promising solution for protein production allowing shorter process times and less protein hydrolysis. One main advantage of CFPS is the ability to express cytotoxic proteins (Khambhati et al., 2019), making a major contribution in the synthesis of difficult to express proteins and particularly TPs. There are many variations of CFPS, composed of either recombinant transcription-translation machinery or derived from prokaryotic or eukaryotic cell lysates. The most utilized lysates are Escherichia coli extract (ECE), rabbit reticulocyte lysate (RRL), Chinese hamster ovary (CHO) lysate, wheat germ (WGE) lysate and insect cell extract (ICE). However, the main CFPS platforms used right now are not completely ideal, with an overall low protein production yield, slow production time, unavailable or inappropriate protein modification and are not feasible, or not sufficiently feasible, for the biosynthesis of TPs. To address some of these issues, Buntru et al. (2014) developed a cell-free system using tobacco BY-2 (bright yellow - 2) cells, which showed a higher protein yield in a shorter period of time. But a HTS for accessible and functional TP drug candidates is still not available.

The complexity and barrier rendered by the cell membrane prompt numerous difficulties, such as experiments being hard to standardize, incompatibility issues, and variability. To overcome the challenges in the biosynthesis of TPs and provision of TP, the prior art uses living cells or artificial components e.g. synthetic lipids in combination with a prokaryotic system (e.g. E. coli extract (ECE)) to achieve correctly folded, modified and stabilized TP. Doing so, the above-mentioned disadvantages cannot be overcome and the biosynthesis is more complex, time consuming and more expensive achieving lower yields of the desired TP.

To address these issues, the object of the present invention is to provide an eukaryotic system, preferably a plant cell-free protein synthesis (CFPS) system, as a key tool that is suitable to work without the use of living cells and without artificial components for the synthesis of correctly folded, modified and stabilized TP. The provision of TPs embedded within a lipid bilayer of endogenous microsomes enables the provision of the inventive HTS platform for ligands as drug candidates of TPs.

Thus, the object of the invention is to provide a method for the synthesis of TPs embedded in a lipid bilayer derived from endogenous microsomes and to provide the TP embedded in said lipid bilayer of interest for a screening for ligands, in particular as drug candidates. Another object is to provide stable and correctly folded TPs of interest embedded in a lipid bilayer derived from endogenous microsomes. Further, it is the aim to provide endogenous microsomes or fragments thereof comprising at least one TP embedded in said lipid bilayer. It is another object of the present invention to provide TPs embedded in said lipid bilayer captured on a surface for subsequent screening and detection methods for/of interaction but still mobile TPs, in particular mobile in a medium of the desired detection method, e.g. in a liquid phase, gel phase or gas phase. Alternatively, another object is to provide captured and immobilized TPs on a device, e.g. on a microtiter plate, microchip, microarray, microfluidic device or other consumables for detection purposes. Thus, it is another object to provide the appropriate consumable for the desired detection method comprising the TP of interest embedded in said lipid bilayer and captured on the consumable. By provision of high yields of lipid bilayer embedded TPs it is the aim to provide a flexible and adaptable Kit for the desired screening design. Another object of the present invention is to provide an optimized and standardized system for a high throughput screening (HTS) for ligands of TP embedded in a lipid bilayer, preferably optimized and standardized for drug screenings. It is an object to enable, improve, standardize and accelerate HTS of drug candidates for TPs which are associated with diseases or with the treatment of diseases e.g. with antibodies, chimeric antigen receptors and the like. For that it is an object to provide a HTS platform that is easily adaptable to any TP of interest.

Another object is to provide a method wherein mild conditions during cell lysate preparation provide intact subcellular membranous structures (microsomes) derived from the ER and/or Golgi. These membrane vesicles are crucial prerequisites for the production of functionally active TP with their subsequent PTM. Thus, another object of the invention is the provision of a method wherein the TP are expressed, folded, modified and stabilized in the microsomes. PTMs strongly affect the physicochemical properties of nascent polypeptide chains, thus influencing protein folding, stability, and activity. It is an object to provide isolated and endogenous membranous structures carrying the TP of interest.

The inventive cell-free system overcomes the aforementioned disadvantages of the prior art and provide fast access to the TP of interest, low reaction volumes, and short reaction times and thereby enables high-throughput TP expression strategies and high-throughput screening with TP. The inventive method provides full-length TPs with correct folding and incorporates co-translational modifications and post-translational modifications (PTMs). Another advantage of the present invention is that it is not necessary to add artificial membrane structures or exogenous microsomes from other species. The advantage of the present invention is that it provides a homogenous CFPS for TP. The methods, kits and assay according to the present invention are suitable to be used for expression and testing of higher libraries, multiplex design, allowing complex studies and high throughput experiments to work sufficiently faster, quicker, and at a lower cost. The details of the invention are explained below.

The first aspect of the present invention is a high-throughput screening (abbreviation HTS) for at least one ligand of at least one transmembrane protein (TP) of interest that is embedded in a lipid bilayer of at least one endogenous microsome derived from plant-based endoplasmatic reticulum (ER), in particular from ER and Golgi, comprising the steps
- providing at least one endogenous microsome - as defined herein - comprising at least one lipid bilayer embedded transmembrane protein (TP) or at least one endogenous microsomal fragment comprising at least one lipid bilayer embedded transmembrane protein (TP),
- providing at least one analyte of interest - as defined herein,
- contacting of the at least one TP with the at least one analyte and
- detection of interaction between the at least one analyte and the at least one TP.

The advantage of an endogenous microsome - as defined herein - derived from plant-based endoplasmatic reticulum (ER) in particular from ER and Golgi, is that it satisfies all the necessary requirements for proper folding of TPs because such microsomes offer a native environment and intact translocon machinery for a proper embedment and folding of TPs. Most preferably the endogenous microsome according to the invention is derived from cultured Tobacco-BY-2 cells. With the inventive HTS, desired activity assays as well as binding assays are possible in order to identify ligands modulating an activity of the TP or in order to identify binding partners of the TP.

In one embodiment the inventive HTS further comprises a step of treating (destruction or disruption) the at least one microsome, in one embodiment the free microsome, to obtain microsomal fragments, in one embodiment free microsomal fragments, wherein the at least one TP remains embedded in the said lipid bilayer derived from the endogenous microsome. A "free microsome" within the meaning of the invention is an endogenous microsome comprising at least one TP embedded in the lipid bilayer of the microsome that is not captured via the TP to a biological non-active surface.

"Treating" the microsomes comprising at least one TP embedded in the lipid bilayer according to the invention means that the whole and intact microsome comprising at least one TP, one or more different TPs or one or more TP variants of the same TP of interest, is subjected to chemical and/or physical forces to break down the lipid bilayer. Chemical and/or physical forces strong enough to rupture (destruct) the lipid bilayer are applied, but as little as possible so as not to affect the conformation and activity of the at least one TP. Chemical forces comprise treating with detergents. Detergents comprise maltosides, Thiomaltoside, Polyoxyethylene, polyoxyethylene glycols, glucosides, Gluconamidopropyl, Thioglucopyranoside, Glucopyranoside, Glucamide, Methylglucamide, Hydroxyethylsulfoxide, detergents from the maltose-NG family e.g. Lauryl Maltose Neopentyl Glycol (LMNG), amine oxides, n-Dodecyl β-D-maltoside (DDM), n-Decyl-β-D-Maltopyranoside (DM), n-Dodecyl-α-D-Maltopyranoside (a-DDM), Octyl-beta-glucoside (OG) and Lauryldimethylamine-N-oxide (LDAO), CYMAL, detergent GDN-101, OGNG, LDAO, UDAO and APO109, Tween 20 and Fos-choline detergents e.g. fos-choline U10-11, cyclofos-6, 12, 13, 14, 15 and 16. Physical forces comprise shear forces (by means of shear force generating devices, different mixer), ultra-wave, sonication, ultrasonic. Any method of treating can be combined. Thus, said treating is a chemical treatment (e.g. detergents) and/or a physical or mechanical treatment (e.g. shear forces, ultrasonic) wherein a force in an extent is exerted on the microsome sufficient to destruct the microsomes but without damaging the TP or without dissolving the TP from the lipid bilayer.

If necessary, microsomal fragments according to the present invention may be stabilized with an excipient. A stabilizing agent or stabilizing excipient is a molecule that is suitable to stabilize the microsome or microsomal fragment comprising at least one TP embedded in the lipid bilayer of said microsome or microsomal fragment. The stabilizing agent ensures that said at least one TP remains folded and embedded within the lipid bilayer and the binding site or epitope of interest remains accessible for the analyte. Stabilizing agent within the meaning of the invention encompasses lipid bilayer and/or protein stabilizing agents which are well known by the skilled person (Sachse et al. 2014). Such excipients comprise so called nanodiscs, nanoparticles, scaffold proteins or any combination thereof. Thus, in another embodiment of the present invention the HTS may comprise a step of adding at least one stabilizing excipient, preferably a lipid bilayer stabilizing excipient, to the microsomal fragment, preferably after a treating step which may be prior or after capturing. The "treating" as described herein does not weaken or destroy the captured state of the at least one TP. However, it may be desirable to perform treating prior to capturing if the elected capturing strategy would be not strong enough to withstand the available or preferred treating method.

The step of treating the at least one microsome may be performed independently (temporal and spatial) from the HTS, it may be a step of the production method (biosynthesis) for said TP described herein according to the invention or a step of the HTS according to invention. It may be desirable to produce the at least one TP in a microsome, to store the intact microsome comprising the at least one TP under suitable conditions to enable individual decision on time and design of HTS. E.g. HTS with intact microsomes at any time or destruction of them at any time in order to provide microsomal fragments immediately before the HTS. In this embodiment it may be desirable or necessary even to store the microsomes or fragments thereof as defined herein. If necessary, a stabilizing excipient may be added.

In a preferred embodiment of the HTS according to the present invention, the at least one microsome or microsomal fragment is derived from the genus Nicotiana of the family Solanaceae. Preferably from Nicotiana tabacum, more preferably from a BY-2 cell line.

Nicotiana is a genus of herbaceous plants and shrubs in the family Solanaceae. So far, at least 67 species are known and are commonly referred to as tobacco plants. Some of them, mostly N. tabacum, are cultivated for tobacco leaves for the production of tobacco products. Generally any species that can be cultivated or of which a cell line can be isolated is feasible within the meaning of the present invention. In particular for the provision of endogenous microsomes according to the present invention. N. tabacum or a cell line derived thereof is the preferred source for microsome or microsomal fragment, more preferably the BY-2 cell line from N. tabacum. In another embodiment of the HTS according to the invention the at least one endogenous microsome or endogenous microsomal fragment derived from plant-based endoplasmatic reticulum (ER), in particular from ER and Golgi, is from a cellular lysate from a plant of the genus Nicotiana of the family Solanaceae, preferably from a cellular lysate of N. tabacum, more preferably a cellular lysate of a BY-2 cell line from N. tabacum.

The BY-2 cell line and BY-2 cell-free protein synthesis (CFPS) were developed to overcome the drawbacks presented by most eukaryotic CFPS systems. Compared to lysates of wheat germ (WGE) for protein production, BY-2 shows a faster lysate production with higher translational activity and higher potential for scaling-up (Buntru et al., 2014). It was shown that by means of a BY-2 lysate a functional full-size antibody can be produced (Buntru et al., (2015)), showing that BY-2 lysate is a potential expression system for biopharmaceutical manufacturing. BY-2 cells comprise microsomes derived from the Endoplasmic Reticulum (ER) and the Golgi of the plant cells. By applying an appropriate method, said microsomes can be prepared and isolated for the purpose of the present invention. This is important because one of the challenges when producing TPs is that they need to be integrated into a lipid bilayer to be synthesized in their correctly folded state (Buntru et al., (2015). The present invention transfers the production of TPs into a HTS platform that allows the screening of multiple drugs against the otherwise intractable TP targets. A HTS derived from BY-2 is also named BY-2-HTS which is a preferred embodiment of the inventive HTS with endogenous microsomes. The advantage of the endogenous microsome is that complications due to the use of artificial components, artificial membranes or other artificial excipients, are avoided and in addition the native protein biosynthesis machinery, in particular the native posttranslational modification capacity, is maintained.

Another embodiment of the HTS according to the present invention, preferably the BY-2-HTS, is that the at least one TP embedded in a lipid bilayer of the endogenous microsome and/or the endogenous microsomal fragment, preferably from N. tabacum, is captured on a biologically non-active surface. In another embodiment of the HTS according to the invention the biologically non-active surface is a surface of a particle comprising micro-particles, nano-particles and magnetic particles, a surface of a device comprising microtiter plates, microfluidic devices, micro arrays, microchips and/or it is any other surface suitable for capturing the at least one TP of interest.

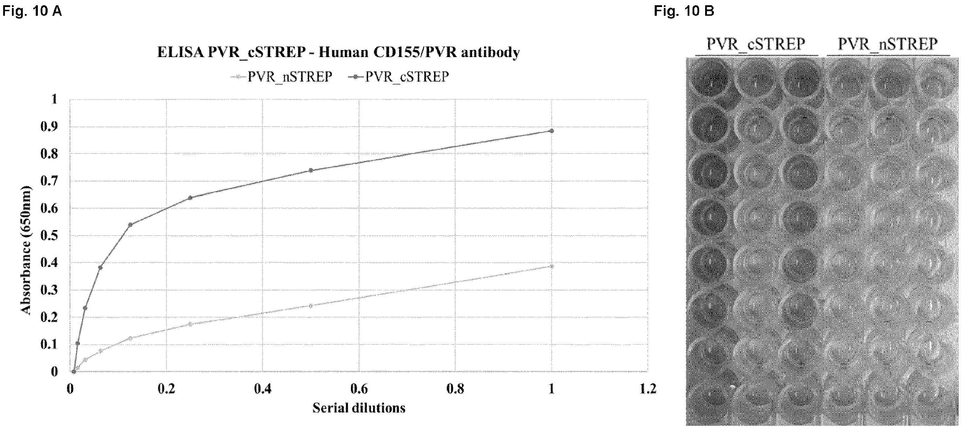

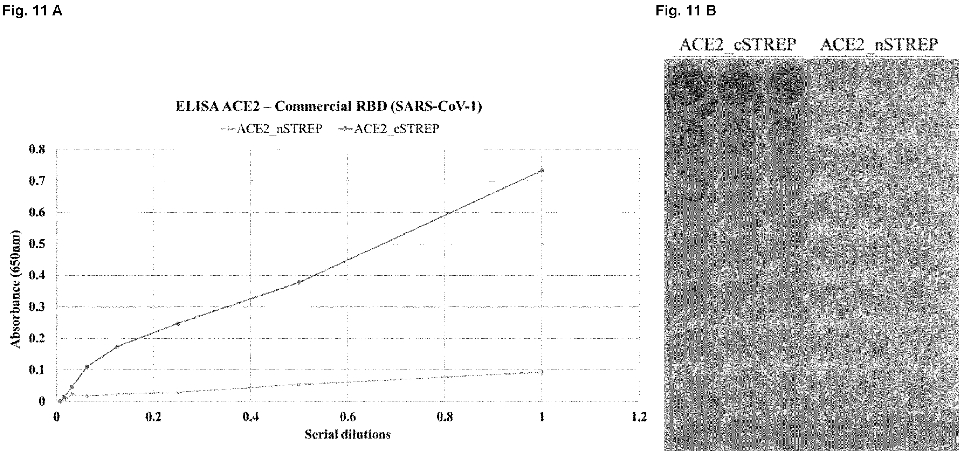

Preferably any other surface that is suitable for capturing, contacting, interaction and detection according to the present invention. Successful capturing, interaction and detection according to the invention is shown in the proof-of-concept example and results are depicted in Fig. 10 and 11.

For all aspects and embodiments of the present invention a transmembrane protein (TP) is "captured" to a biological non-active surface so that said TP is under control during interaction and/or detection of said interaction. "Capturing" according to the invention may be performed by non-specific binding of the at least one TP to the biological non-active surface or capturing is mediated by at least one tag at the C-terminus and/or N-terminus of the at least one TP. Preferably the at least one TP is captured on a biologically non active surface via the C-terminal tag. Suitable tags are well known to the skilled person and comprise Biotin tags, Hapten-tags, Alkaline Phosphatase (AP), AU1 epitope, AU5 epitope, Bacteriophage T7 epitope (T7-tag), Calmodulin binding peptide (CBP), Cellulose binding domain (CBP), Chitin binding domain (CBD), Chloramphenicol Acetyl Transferase (CAT), Choline-binding domain (CBD), E2 epitope, FLAG epitope, Galactose-binding protein (GBP), Glu-Glu (EE-tag), Glutathione S-transferase (GST), HaloTag^{®}, Histidine affinity tag (HAT), HSV epitope, Human influenza hemagglutinin (HA), KT3 epitope, LacZ, Maltose-binding protein (MBP), Myc epitope, PDZ domain, PDZ ligand, Polyarginine (Arg-tag), Polyaspartate (Asp-tag), Polycysteine (Cys-tag), Polyhistidine (His-tag), Polyphenylalanine (Phe-tag), Profinity eXact, Protein C, S1-tag, S-tag, Staphylococcal protein A (Protein A), Staphylococcal protein G (Protein G), Strep-tag, Strep-tag II, Streptavadin, Streptavadin-binding peptide (SBP), T7 epitope, Tandem Affinity Purification (TAP), TrpE, Universal and VSV-G. A "captured TP" is always embedded in a lipid bilayer of an endogenous microsome or of a microsomal fragment as defined herein. Thus "captured TP" is synonymously used for "captured TP embedded in a lipid bilayer of an endogenous microsome or microsomal fragment". "captured TP" means that the TP embedded in a lipid bilayer is fixed on the at least one biologically non active surface achieved by the capturing step and said surface is carrying the TP during the step of treating, contacting, interaction and/or detection of the HTS, preferably of the BY-2-HTS.

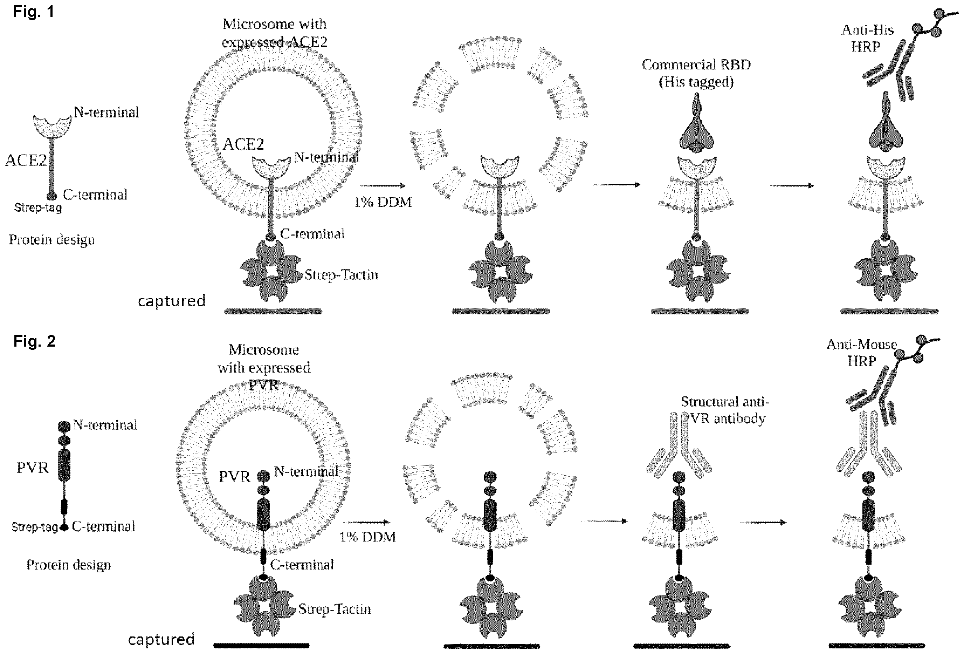

Thus, in another embodiment of the inventive HTS, preferably of the BY-2-HTS, the at least one TP comprises at least one tag at the C-terminus and/or N-terminus, preferably the at least one TP is captured on a biologically non-active surface via the C-terminal tag. Any tag is suitable. A Strep-tag, His-tag and/or Biotin-tag are preferred (Fig. 1-5).

In one embodiment the at least one microsome comprising the at least one lipid bilayer embedded TP is captured first and afterwards the microsomes are treated as described herein to obtain microsomal fragments comprising at least one lipid bilayer embedded TP that is captured during and after treatment of the at least one microsome. Alternatively the at least one microsome is treated in a non-captured state, preferably free state, to achieve said fragments comprising at least one lipid bilayer embedded TP and afterwards said fragments are captured via the at least one TP to said biological non active surface. If desired, microsomal fragments comprising the at least one TP are separated from fragments without TPs and/or from dissolved TPs.

Thus, in another embodiment the HTS according to the invention, preferably of the BY-2-HTS, further comprises the steps
- capturing of the at least one TP embedded within a lipid bilayer of a microsome on the at least one biologically non-active surface, and
- treating the at least one captured microsome to obtain microsomal fragments, wherein the at least one TP remains embedded in a lipid bilayer, or alternatively
- treating the at least one free microsome to obtain free microsomal fragments, wherein the at least one TP remains embedded in a lipid bilayer, and
- capturing of the at least one TP embedded within a lipid bilayer of a microsomal fragment on the at least one biologically non active surface

Preferably said treating is a chemical treatment (e.g. detergents) and/or a physical treatment (e.g. shear forces, ultrasonic) in an extent sufficient to destruct the microsomes without damaging the TP within the lipid bilayer. Preferably capturing is performed with a Strep-tag, His-tag and/or Biotin-tag. If desired non captured microsomes and/or microsomal fragments are separated after the capturing step. Microsomes and/or microsomal fragments without TPs and/or dissolved TPs are separated from Microsomes and/or microsomal fragments comprising the at least one TP. Separation may be performed by filtration and/or centrifugation.

The biological non-active surface may be a surface of a particle, of a device as defined herein and/or any other surface suitable for capturing the at least one TP of interest. In any case a "captured TP" is captured within a reaction zone of the aforementioned surfaces wherein the interaction between the at least one TP and the at least one analyte is detected. Thus, a "biological non active surface" according to the invention means any surface that does not influence or affect the step of contacting, the interaction between the at least one TP and the least one analyte and/or detection of said interaction. The biological non active surface may be a surface of a material in particular usually used for consumables of any detection assay, e.g. polymers, polystyrene, polypropylene, polycarbonates, alginate, agarose, hydrogels, alginate or Agarose hydrogels, polycarboxylate, carboxy methyl dextran hydrogels, glass material etc.. Other materials suitable for a biological non-active surface are well known to the skilled person. The biological non-active surface is a surface of a particle comprising micro-particles, nano-particles and magnetic particles, a surface of a device comprising microtiter plates, microfluidic devices, micro arrays, microchips or any other consumable and/or of any other surface suitable for capturing and to carry the TP of interest in particular during the step of contacting, interaction and/or detection. Said surface is in particular suitable to capture and to carry the at least one TP during the HTS according to the present invention, in particular during treating of the at least one microsome, during the step of contacting, interaction and/or detection of interaction, preferably binding, of the at least one TP with the at least one analyte without affecting said steps.

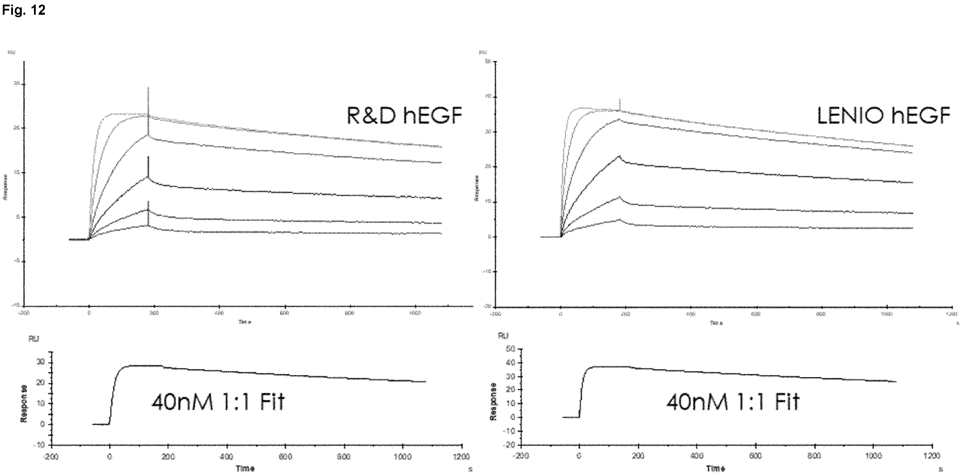

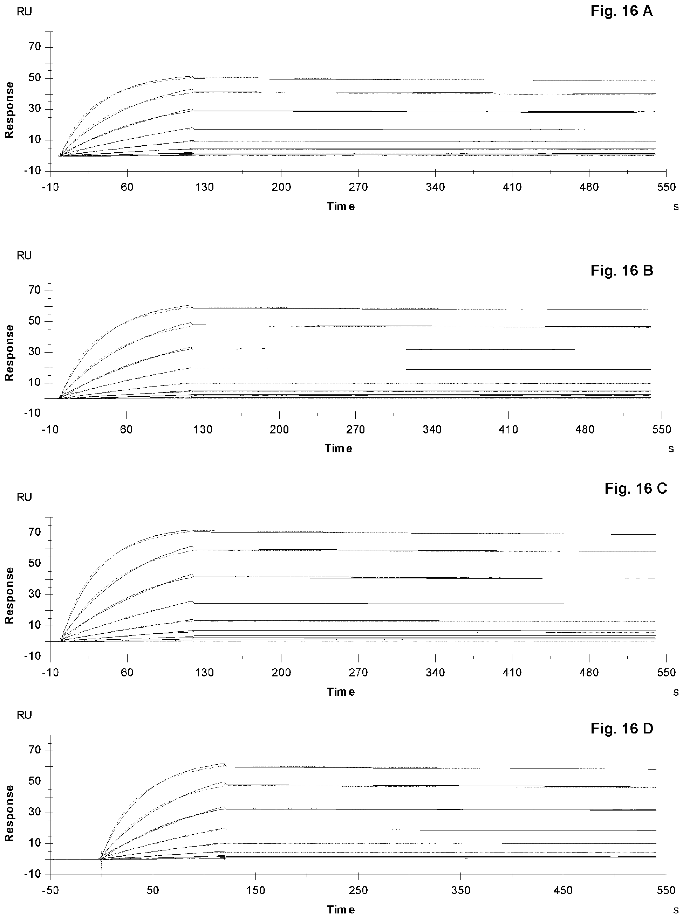

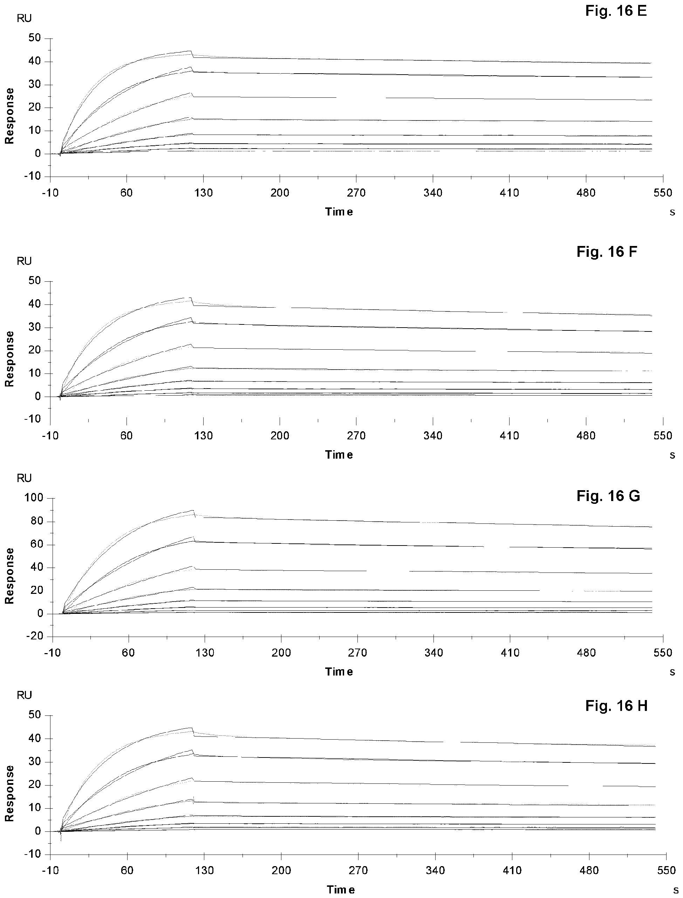

A device within the meaning of the present invention comprises, is coated with or consists of a biological non-active surface, preferably the device comprises at least two, three, four, five, six, seven, eight, nine, 10, up to 16, at least 16 up to 24, at least 24 up to 32, at least 32 up to 96, at least 96 up to 384, at least 384 up to 1536 reaction zones as defined herein or any other integer between the aforementioned ranges. A microtiter plate typically has 6, 12, 24, 48, 96, 384 or 1536 wells usually arranged in a 2:3 rectangular matrix. Devices and particles - as defined herein - are consumables, preferably suitable for use in the HTS according to the invention and most preferably for the step of capturing, treating, contacting, interaction and/or detection of said interaction. An example of such a device has been successfully used in the proof-of-concept example and results achieved with said device are depicted in Fig. 10 and 11 and proof that said device, here a microtiter plate, is feasible according to the present invention. In particular said device is feasible for use in the HTS according to the present invention, preferably BY-2-HTS, more preferably multiplex BY-2-HTS, and it can be produced by the cell-free production method, in particular wherein the TP embedded in a lipid bilayer is captured to a device according to the present invention. The same applies for a microfluidic device (Fig. 12 and 16).

Thus, another aspect of the invention is a device - as defined herein - comprising at least one biologically non active surface - as defined herein -, respectively carrying the at least one captured TP - as defined - that is embedded in a lipid bilayer of a microsome or of a microsomal fragment - as defined herein. The device comprises at least two or more reaction zones comprising a biologically non-active surface respectively for use in the multiplex HTS, preferably multiplex BY-2-HTS. In one embodiment the HTS, preferably BY-2-HTS, comprises the step of
- Provision of at least one device comprising at least two reaction zones comprising at least one biologically non-active surface, and
- capturing - as described herein - of the at least one TP embedded within a lipid bilayer of a microsome in each reaction zone to the at least one biologically non-active surface of said device, and optionally treating to obtain microsomal fragments as defined herein or
- capturing - as described herein - of the at least one TP embedded within a lipid bilayer of a microsomal fragment in the at least one reaction zone to the at least one biologically non-active surface of said device.
- Alternatively, provision of at least one device comprising at least two reaction zones comprising at least one biologically non-active surface on which the at least one TP is already captured, wherein said TP is embedded within a lipid bilayer of a microsome or microsomal fragment, and
- provided that it is a captured microsome in the device and a microsomal fragment is desired for HTS, a step of treating follows as described herein, or
- provided that it is captured microsomal fragment in the device the other steps of the inventive HTS are performed comprising provision of at least one analyte or an analyte panel, contacting, interaction and/or detection.

If desired the TP embedded in the lipid bilayer and captured within a device according to the invention may be covered with a stabilizing agent during storage prior to performing of the HTS.

A "reaction zone" according to the present invention is an area wherein the TP is captured and wherein the contacting with the least one analyte takes place and wherein interaction with the at least one analyte is tested. A reaction zone comprises, is coated with or consists of a biological non-active surface as defined herein. The reaction zone is any particle surface of any particle defined herein or located on said particle. The reaction zone is part of a surface of a device as defined herein of a well of a microtiter plate, of a channel of a SPR consumable, of any microfluidics, of any microfluidic device or of any other surface of consumables used in the detection according to the invention. A device comprises at least two or more of such reaction zones comprising, coated with, or consisting of a biological non-active surface as defined herein.

In another embodiment of the HTS according to the present invention, preferably of the BY-2-HTS, the at least one analyte comprises a small molecule, peptide, polypeptide, soluble protein, membrane protein, protein complex or any combination of the aforementioned.

An "analyte" within the meaning of the invention and of any aspect and embodiment means any molecule(s) that is tested for an interaction as defined herein with the at least one TP of interest, preferably tested for its capacity to modulate activity (preferably due to a signalling or signal transduction as defined herein) of the at least one TP, for binding to a specified domain of the least one TP and/or of a binding domain or an epitope on the at least one TP. An analyte may be tested alone or in combination with or dependent on another analyte of the same group (e.g. one, two or more small molecules) or of another group (e.g. a small molecule and any protein). Thus, two or more analytes tested in the same reaction zone are considered as "analyte complex" ("analyte combination" synonym) and are tested in combination for any interaction with the at least one TP of interest. Thus the present screening is suitable to screen at least one "analyte" or an "analyte complex" (analyte combination, synonym) for its potential to being a ligand according to the present invention. Examples for an analyte complex comprise a multimeric protein comprising at least two polypeptide chains, at least two analytes combined by covalent or electrostatic forces or hydrogen bonds and at least one analyte combined with a polymer, nanoparticle, colloid or liposome. An analyte is tested for its capacity to induce a signalling or signal transduction due to the interaction with the least one TP, preferably binding of the analyte to the TP. Said signalling or signal transduction (preferably induced due to binding of the at least one analyte to the least one TP) may occur through the at least one transmembrane domain of the least one TP (in particular towards the originally cytosolic domains of the TP, that in the assay is preferably captured to a biologically non-active surface) or at the same side of interaction of the least one TP with the at least one analyte. Said signalling or signal transduction may modulate a biological process and modulation comprises activation, deactivation, inhibition, enhancement, induction or blocking of a biological process. Thus, the effect of the analyte identified as an ligand can be that of an activator, inhibitor, effector, trigger or modulator of an biological process. Preferably, of a biological process associated with a disease. An "Analyte" within the meaning of the invention means any molecule(s) as explained above, that preferably is/are a native molecule(s) of microbial, bacterial, fungal, viral, plant, animal and/or mammalian origin. Analyte encompasses small molecules, peptides (e.g. Dipeptide, Tripeptide, Tetrapeptide, Pentapeptide, Hexapeptide, Heptapeptide, Octapeptide, Nonapeptide, Oligopeptide), polypeptides, soluble proteins, membrane proteins, and/or a protein complex of microbial, bacterial, fungal, viral, plant, animal and/or mammalian origin. Analyte within the meaning of the invention comprises antibodies, antibody fragments, fc fragments, fab fragments, Immunoglobulin (e.g. A, G, D, E, F, M, W, Y), Ig fragments, regulatory proteins comprising inhibitors, enhancers, antagonists, membrane proteins, antigens, antigen fragments, chimeric antigen receptors, fragments thereof, receptors, cytokine receptors and/or the analyte is any protein produced by the use of ALICE^{®}. An "analyte panel" comprise two or more analytes or analyte combination for the multiplex HTS, preferably multiplex BY-2-HTS, according to the invention.

"Small molecules" are defined differently depending on the technical field of application. Presently small molecules or micromolecules (synonym) have a low molecular weight (≤ 1000 Daltons) and/or low *molecular mass* of less than 900 g/mol. They may regulate a biological process, are of organic origin, may comprise secondary metabolites as well as small nucleic acid molecules comprising deoxynucleotide monophosphates and small molecule oligonucleotide. Some examples for small molecules comprise antihistamines, antibiotics, drug candidates and small molecules of microorganisms and plants, selective serotonin reuptake inhibitors, alicylic acid, ibuprofen, paracetamol, morphine, ethanol, psilocybin, L-DOPA, cyclophosphamide, methotrexate, 5-fluorouracil, vinorelbine, doxorubicin, cyclophosphamide, docetaxel, bleomycin, vinblastine, dacarbazine, mustine, vincristine, procarbazine, prednisolone, etoposide, cisplatin, epirubicin, capecitabine, folinic acid, oxaliplatin, gemcitabine, ifosfamide, hydrocodone, metformin, losartan, albuterol, gabapentin, omeprazole, levothyroxine, atorvastatin, brompheniramine, cetirizine, chlorpheniramine, clemastine, diphenhydramine, fexofenadine, loratadine, amoxicillin, doxycycline, cephalexin, ciprofloxacin, clindamycin, metronidazole, azithromycin, sulfamethoxazole, trimethoprim, levofloxacin, citalopram, escitalopram, fluoxetine, paroxetine, sertraline.

A "ligand" within the meaning of the invention is any analyte that interacts with the at least one TP within the meaning of the present invention, preferably that binds to the at least one TP. Therefore an "analyte" (or analyte complex) within the meaning of the invention that has a proven capacity to induce a signalling or signal transduction due to the interaction with the least one TP, preferably binding of the analyte to the TP, is identified as a ligand. Preferably, due to said interaction, preferably binding to the at least one TP, a desired biological process is modulated, preferably a biological process that is associated with a diseases. Most preferably the disease is associated with the tested TP. More preferably because of said modulation as defined herein the analyte that is a ligand to the tested TP is considered as a drug candidate. The desired capacity of the at least one ligand is suitable for a prevention or treatment of the disease associated with the at least one TP. The Ligand may be one analyte or an analyte complex as defined herein and proven by a detected interaction according to the invention.

"Interaction" between the at least one TP and the at least one analyte (or analyte complex) within the meaning of the present invention, in particular after contacting, is a molecular interaction that cause a measurable activity or change of activity of the at least one TP that is induced by the at least one analyte and/or interaction is a binding between of the at least one TP and the at least one analyte. Said binding can be associated with a measurable activity, change of activity or not. In general, the interaction may cause modulation of an activity - increase, decrease - that is detected as a change of activity because of the analyte's capacity to modulate said activity as defined herein. Consequently, within the meaning of the invention the interaction is proven by detection of an activity, such as a colori-/fluorimetric assay, radiometric scintillation proximity assay, atomic force microscopy (AFM), spectrophotometry, mass spectrometry, Raman spectroscopy, membrane potential assay, electrochemical assay, Fluorescence Resonance Energy Transfer (FRET) assay, crystallography, cryogenic electron microscopy. The binding may be proven by detection of the analyte bound to the TP or by detection of an activity after binding. Interaction may be performed and detected in a multiplex screening.

Thus, another aspect of the inventive HTS is a multiplex high-throughput screening (multiplex HTS), preferably multiplex BY-2-HTS, for at least one ligand of at least one TP of interest providing two or more analytes in two or more reaction zones simultaneously and/or providing an analyte combination of two or more analytes simultaneously in two or more reaction zones. All definitions apply accordingly for the multiplex design of the inventive HTS.

"Multiplex", "multiplex screening", "multiplex HTS" or "multiplex high-throughput screening" (synonym) means that in the same reaction zone one or more different TPs are captured to a biological non-active surface and are tested against one analyte or a defined analyte combination and interaction of at least one or more captured TPs with the analyte or the analyte combination is detected. Multiplex means also that in different reaction zones the same TP is captured to a biological non active surface and each TP is tested against two or more different analytes and in each reaction zone the interaction between the one captured TP and the one analyte or an analyte combination is detected. Thus "multiplex" within the meaning of the invention means that for the at least one or more TP of interest at least two, three, four, five, six, seven, eight, nine, 10, up to 16, at least 16 up to 24, at least 24 up to 32, at least 32, at least 96 up to 384 analytes or more, or analyte combinations are screened simultaneously. Or any other integer between the aforementioned ranges. All other definitions apply accordingly and any step and combination of steps comprising, providing the microsomes or microsomal fragments, providing of analytes treating, capturing, contacting, interaction and/or detection and described embodiments of the invention may be performed in a multiplex HTS, preferably multiplex BY-2-HTS, as described herein. A multiplex HTS can be performed with any embodiment of the biological non-active surface, device or consumable - as defined herein respectively.

The multiplex grade is defined or limited by the available or elected detection method. In particular, by the available consumables for use in said detection method, e.g. microtiter plates, microfluidic devices, microchips, microarrays or consumables for SPR.

Thus, an embodiment of the multiplex HTS, preferably multiplex BY-2-HTS, comprise the steps
- providing a plurality of endogenous microsomes each comprising at least one lipid bilayer embedded transmembrane protein (TP) or a plurality of endogenous microsomal fragment each comprising at least one lipid bilayer embedded transmembrane protein (TP),
- providing a plurality of analytes or of analyte combinations - as defined herein,
- contacting a plurality of TPs with a plurality of analytes or of analyte combinations, and
- detection of interaction of any one of the analytes and of any of the TPs, preferably in a plurality of reaction zones of a device as defined herein, more preferably in a microtiter plate, in a microfluidic devices or in a SPR consumable.

The steps of capturing and/or treating as described for the HTS apply accordingly for the multiplex design.

In another embodiment of the HTS, preferably multiplex BY-2-HTS, according to the present invention the interaction (as defined herein) between the at least one TP and the at least one analyte, in particular after contacting, is detected by means of an immunoassay, preferably enzyme-Linked immunosorbent assay (ELISA), a spectroscopic assay, preferably Surface Plasmon Resonance (SPR), a particle size measurement, a magnetic measurement and/or an optical measurement. Such methods are well known to the skilled person. In the experiments the inventive HTS combined with an ELISA and SPR is shown. The methods are well known to the skilled person and it is clear to the skilled person that either the analyte or the at least one TP or both comprises means for detection or that such means have to be added separately in order to detect any interaction. Means for detection comprise fluorescent molecules, tags, Hapten, enzymes or other molecules allowing detection of interaction. Such means may be encoded by the template encoding for the at least one TP, bound to the at least one analyte and/or may be added separately in the step of detection. Appropriate means for detection are well known for each of the mentioned detection methods and feasibility is proven in the presented examples.

In one embodiment of the present invention the interaction and/or detection, in particular after contacting, of HTS is performed with an immobilized or with a mobile microsome or microsomal fragment respectively each comprising at least one TP embedded within a lipid bilayer that is captured to a biological non-active surface. All definitions apply accordingly. However, "captured" is not the same as "immobilized" and "captured and mobile" and "captured and immobilized" have to be distinguished. A microsome or microsomal fragment may be captured via the at least one TP and said microsome or microsomal fragment is mobile or immobilized. Contrary to the mobile status using a biologically non-active surface of a particle as defined herein the microsome or microsomal fragment is immobilized within a device by the step of capturing the at least one TP on the at least one biologically non-active surface.

As described herein, TPs are highly interesting for drug development and constitute the majority of current drug targets. Nevertheless, TPs are hard to express proteins that are poorly characterized. The BY-2 lysate, preferably the commercially available ALiCE^{®} system (LenioBio GmbH, e.g. ALiCE^{®} Kit AL0103050, AL0103200, AL0103500) was presently used to show overexpression and production of TPs, and preforming the HTS according to the present invention. Presently a binding assays against its known ligands was tested in order to show proof of concept for the HTS platform, preferably for a By-2-HTS, according to the present invention. For this, the well-characterized type I transmembrane glycoproteins and their ligands were used. These proteins are Angiotensin converting enzyme (ACE2), T-cell receptor with Ig and ITIM domains (TIGIT) and its respective ligands, the receptor binding domain of the spike protein of SARS-CoV-1/2 (RBD) and poliovirus receptor (PVR). These TPs are of interest because they have shown to be "druggable" targets and they are involved in very important current diseases, like COVID-19 and cancer. Hampering the binding between the TP in question and its ligands could effectively act as a possible therapy. It was shown that the HTS of the present invention is suitable to study different "hits" that hinder the binding. Consequently, proof of concept of the inventive HTS platform to test for new drugs has been shown.

Another aspect of the present invention is a cell-free production of at least one transmembrane protein (TP) of interest that is embedded in a lipid bilayer of at least one endogenous microsome derived from plant-based endoplasmatic reticulum (ER), in particular from ER and Golgi, , preferably based on a plant from the genus Nicotiana of the family Solanaceae, preferably a cell-free protein synthesis (CFPS) system based on BY-2, comprising the steps
- providing a lysate from a plant comprising at least one endogenous microsome, preferably from a plant of the genus Nicotiana of the family Solanaceae, preferably a lysate from N. tabacum, more preferably a lysate of a BY-2 cell line from N. tabacum,
- providing at least one template encoding for the at least one transmembrane protein (TP),
- incubation of the at least one lysate with the at least one template,
- expression of the at least one transmembrane protein (TP),
- coupled transcription and co-translational translocation of the at least one expressed TP into the at least one microsome, and
- obtaining the at least one transmembrane protein (TP) embedded in a lipid bilayer of the at least one endogenous microsome, and
- optionally treating the at least one endogenous microsome carrying the at least one TP embedded in a lipid bilayer to obtain microsomal fragments comprising the at least one TP embedded in a lipid bilayer. If desired the step of
- capturing - as defined herein - to a biologically non-active surface is performed after or prior treating.

The immediately achieved process product is a TP embedded in a lipid bilayer of an endogenous microsome or of an endogenous microsomal fragment which respectively is free (without capturing) as defined herein or a TP embedded in a lipid bilayer of an endogenous microsome or of an endogenous microsomal which respectively is captured and mobile (e.g. on a particle) or captured and immobilized (e.g. on a device) as defined herein.

Optionally further steps are necessary and may be combined with the method described herein, such as separation and/or isolation of said microsomes or said microsomal fragments, purification, washing and/or any other steps necessary to provide the at least one TP embedded in a lipid bilayer of at least one endogenous microsome or endogenous microsomal fragment. Such steps are well known to the skilled person. All embodiments of the step capturing (with or without a tag as defined herein), treating separation and isolation described for the HTS apply for the production method accordingly.

In one embodiment of the cell-free production the step of treating is performed timely independent from the preceding steps. It may be desirable to produce the TP in microsomes, to store the intact and free or intact and captured microsome (mobile or immobilized) and to treat them in order to obtain fragments thereof immediately before the HTS according to the present invention which may be later than the cell-free production of the at least one TP.

Another embodiment of the cell free production (CFP) is the production of at least one captured endogenous microsome derived from plant-based endoplasmatic reticulum (ER), in particular from ER and Golgi, comprising at least one transmembrane protein (TP) of interest that is embedded in a lipid bilayer of said microsome, comprising the steps
- providing a lysate from a plant comprising at least one endogenous microsome, preferably from a plant of the genus Nicotiana of the family Solanaceae, preferably a lysate from N. tabacum, more preferably a lysate of a BY-2 cell line from N. tabacum,
- providing at least one template encoding for the at least one transmembrane protein (TP),
- incubation of the at least one lysate with the at least one template,
- expression of the at least one transmembrane protein (TP),
- coupled transcription and co-translational translocation of the at least one expressed TP into the at least one microsome, and
- obtaining the at least one transmembrane protein (TP) embedded in a lipid bilayer of the at least one endogenous microsome,
- optionally separation, preferably by means of centrifugation, of the obtained at least one microsome comprising the at least one TP of interest as defined above, and
- capturing (as defined herein) of at least one microsome comprising the at least one TP of interest as defined, preferably capturing said microsome to a biologically non-active surface as defined herein, and
- obtaining at least one captured microsome comprising the at least one TP of interest as defined, preferably captured to a particle or a device as defined herein.

Another embodiment of the cell free production provides at least one captured endogenous microsomal fragment derived from plant-based endoplasmatic reticulum (ER), in particular from ER and Golgi, comprising at least one transmembrane protein (TP) of interest that is embedded in a lipid bilayer of said microsomal fragment, wherein further the step of treating the at least one endogenous microsome carrying the at least one TP embedded in a lipid bilayer is performed in order to obtain microsomal fragments comprising the at least one TP embedded in a lipid bilayer. All embodiments of capturing (with or without a tag as defined herein), treating or combinations thereof as described for the HTS of the first aspect of the present invention apply for the cell free production accordingly. Thus cell free production according to the present invention may comprise capturing and/or treating in order to obtain a particle, a device or another consumable comprising at least one captured endogenous microsome or endogenous microsomal fragment derived from plant-based endoplasmatic reticulum (ER), in particular from ER and Golgi, comprising at least one transmembrane protein (TP) of interest that is embedded in a lipid bilayer of microsome or that is embedded in a lipid bilayer of a microsomal fragment.

In another embodiment of the cell-free production or of the cell-free production method the lysate further comprises plastids, chloroplasts, mitochondria, or chloroplasts and mitochondria. In a further embodiment of the cell-free production or of the cell-free production method the at least one microsome or microsomal fragment is derived from the genus Nicotiana of the family Solanaceae, preferably from N. tabacum, more preferably from a cell line of BY-2. If necessary, said method may comprise a step of addition of a composition comprising biologically active microsomes and/or biologically active mitochondria. *Preferably such a composition is* derived from the genus Nicotiana of the family Solanaceae, preferably from N. tabacum, more preferably from a cell line of BY-2.

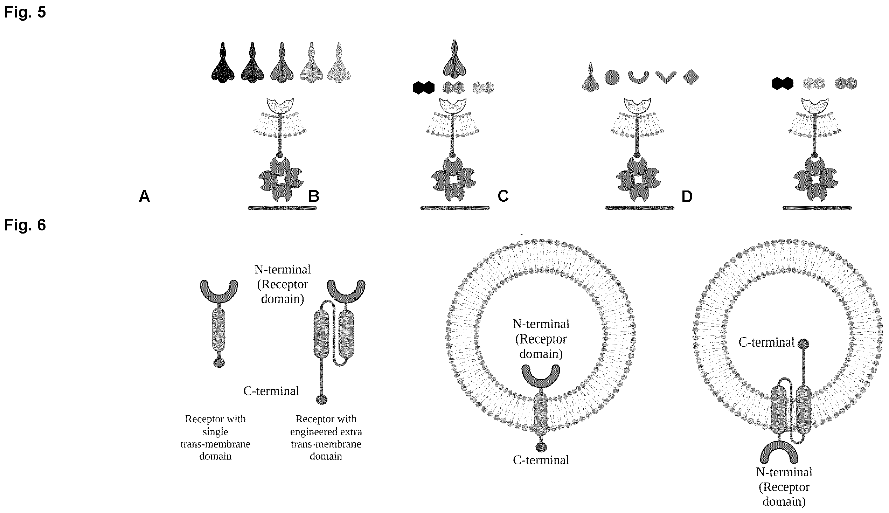

The co-translational translocation into the at least one microsome of the at least one expressed TP during the coupled transcription and co-translational translocation may occur without a signal peptide of the expressed TP or the co-translational translocation is mediated by at least one appropriate signal peptide. Thus, in one embodiment of the cell-free production or of the cell-free production method the at least one template encodes for the at least one TP and for a signal peptide that is suitable to mediate the translocation of the TP into the microsome and that is expressed with the TP from the same template. Other TPs are translocated without the need that translocation is mediated by a signal peptide. In another embodiment the template further encodes at least one tag at the N-terminus and/or at the C-terminus of the at least one TP, preferably the template encodes at least for at least one tag at the C-terminus (Fig. 1-5). Preferably, the TP is expressed with a signal peptide and/or with a at least one tag at the N-terminus and/or at the C-terminus. In another embodiment of the cell-free production or of the cell-free production method, the at least one TP is co-translationally translocated with its C-terminus outside and N-terminus inside of the at least one microsome (Fig. 6). Preferably, glycosylation, N-glycosylation of the TP, other posttranslational modification, lipid modification and/or folding occurs during the step of coupled transcription and co-translational translocation and ensures correctly folded and functional TP. More preferably said TP is embedded with is transmembrane domains within said lipid bilayer as it natively occurs.

Another aspect of the present invention is an endogenous microsome or at least one endogenous microsomal fragment derived from plant-based endoplasmatic reticulum (ER), in particular from ER and Golgi, comprising at least one lipid bilayer embedded transmembrane protein (TP), preferably obtained by the cell-free production or of the cell-free production method according to the present invention. In one embodiment of this aspect, the endogenous microsome or at least one endogenous microsomal fragment is free, not captured and not immobilized. In another embodiment, the endogenous microsome or at least one endogenous microsomal fragment is captured to a particle - as defined herein - but still mobile and still suitable for mixing with at least one stabilizing agent and optionally at least one other excipient to form a composition. In a preferred embodiment, the endogenous microsome or the at least one microsomal fragment is derived from the genus Nicotiana of the family Solanaceae, preferably from N. tabacum, more preferably from a cell line of BY-2. Another aspect of the invention is the microsome or microsomal fragment as described herein with at least one stabilizing agent. Thus, another aspect of the invention is a composition comprising at least one - free or captured and mobile - microsome or microsomal fragment according to the invention, at least one stabilizing agent and optionally at least one other excipient.

Another aspect of the invention is the use of the microsome or microsomal fragment comprising at least one lipid bilayer embedded transmembrane protein (TP) as defined herein in the HTS according to the invention or according to any embodiment of the HTS. All embodiments of capturing (with or without a tag as defined herein), treating or combinations thereof as described for the HTS apply for the use accordingly.

Another aspect of the present invention is a biological non-active surface comprising at least one captured TP of interest that is embedded in a lipid bilayer of at least one endogenous microsome or of at least one endogenous microsomal fragment derived from plant-based endoplasmatic reticulum (ER), in particular from ER and Golgi, wherein the biologically non-active surface is a surface of a consumable. Preferably the consumable is a device comprising two or more reaction zones comprising, consisting of or coated with a biologically active surface as defined herein. Preferably, the device is a microtiter plate, microchip, microarray or microfluidic device, more preferably a microfluidic device (e.g. for SPR) or a microtiter plate (e.g. for ELISA). Another aspect is a consumable as defined herein, preferably a device, comprising at least one captured TP of interest that is embedded in a lipid bilayer of at least one endogenous microsome or of at least one endogenous microsomal fragment derived from plant-based endoplasmatic reticulum (ER), in particular from ER and Golgi. The device comprises at least two or more reaction zones comprising, consisting of or coated with a biologically non-active surface. It is suitable for use in the multiplex HTS according to the present invention. Preferably, said microsome or microsomal fragment is from a plant of the genus Nicotiana of the family Solanaceae, preferably from N. tabacum, more from a lysate from N. tabacum, most preferably from a lysate of a BY-2 cell line from N. tabacum. Capturing is preferably mediated by a tag as defined herein.

For all aspects and embodiments of the present invention a "consumable" comprise, consists of or is coated with a biological non-active surface. Such consumables are used for any detection assay according to the invention. An appropriate material for such consumables comprises polymers, polystyrene, polypropylene, polycarbonates, alginate, agarose, hydrogels, alginate or Agarose hydrogels, polycarboxylate, carboxymethyl dextran hydrogels, glass material etc. Other materials suitable for a biological non-active surface are well known to the skilled person. The definition of the biological non-active surface applies accordingly. Consumable comprise particles, micro-particles, nano-particles, magnetic particles, and devices comprising microtiter plates, microfluidic devices, micro arrays and microchips.

Preferably, said consumable is for use in the HTS, BY-2-HTS, more preferably multiplex BY-2-HTS, or any embodiment thereof according to invention. More preferably, said consumable is produced by the production method as defined herein. More preferably, said consumable is for use during contacting, in the step of treating, interaction and/or detection of interaction between the at least one analyte and the at least one TP according to the invention.

Another aspect of the present invention is a kit for use in the a high-throughput screening (HTS) according to the present invention, preferably BY-2-HTS, more preferably multiplex BY-2-HTS, for at least one ligand of at least one transmembrane protein (TP) of interest that is embedded in a lipid bilayer of at least one endogenous microsome derived from plant-based endoplasmatic reticulum (ER), in particular from ER and Golgi, preferably derived from a plant of the genus Nicotiana of the family Solanaceae, preferably from N. tabacum, preferably from a lysate of N. tabacum, more preferably from a lysate of a BY-2 cell of N. tabacum, comprising
- a plant cellular lysate comprising endoplasmatic reticulum (ER), in particular from ER and Golgi, derived endogenous microsomes preferably from a plant of the genus Nicotiana of the family Solanaceae,
- at least one vector encoding for a known TP as positive control, preferably as a positive control for a functional microsome or microsomal fragment,
- optionally at least one vector as positive control for lysate function
- at least one vector for at least one template encoding for the at least one TP of interest
- optionally further excipients, preferably comprising small molecule building blocks, labelled amino acids, cofactors etc.,
- optionally at least one agent, preferably at least one mean, for detection of the interaction between the at least one analyte and the at least one TP, preferably antibodies for detection
- optionally at least one agent, preferably a suitable detergent, for destruction of the at least one microsome and
- optionally at least one analyte or an analyte panel of two or more analytes.

The kit may comprise additional components such as a composition comprising biologically active microsomes and/or mitochondria as defined herein. Another further component may be a consumable for the capturing as defined herein. Alternatively, the kit comprises a consumable or a device according to the present invention already comprising at least one captured TP. The Kit may comprise at least one instruction specifying the admixture of components and/or an instruction specifying the HTS and/or the cell-free production method, respectively with or without capturing and/or treating.

Preferably, the at least one positive control for lysate function is the a commercially available pALiCE01-eYFP vector, wherein eYPP stands for enhanced yellow fluorescent protein and the at least one positive control for functional microsomes or microsomal fragments is the pALiCE0X-TP vector for TP expression, preferably customized or adaptable to the TP of interest. However, any other vector and/or vector and TP combination is suitable provided that it allows a confirmation of the desired function (positive control).

A preferred embodiment of the Kit for use in the HTS, preferably multiplex HTS, further comprises
- further excipients, preferably comprising small molecule building blocks, labelled amino acids, cofactors etc.,
- agents for detection, preferably antibodies for detection,
- optionally agents for destruction of microsomes, and
- optionally at least one analyte or an analyte panel of two or more analytes

A preferred embodiment of the Kit for use in the HTS, preferably multiplex HTS, further comprises
- agents for chemical destruction of the inventive microsomes, and
- optionally at least one analyte or an analyte panel of two or more analytes

For all aspects and embodiments of the present invention, an "endogenous microsome" means that it is not artificial and that it is from a cell free production system of an eukaryotic species. Preferably, it is from the same system from which the lysate originates from and where the ER, Golgi, microsomes, plastids, chloroplasts and/or mitochondria are from. A "lysate" is the isolated biological active content for protein synthesis as it is known from the prior art. An "endogenous microsome" is from a native system derived from only one species of the same family. A native lysate may comprise components from different species and/or different genus but originates from the same family. Thus, different cell-lines of the genus Nicotiana, different cell-lines of N. tabacum and different BY-2 cell lines with different genetic modifications respectively may be combined. A microsome mixture derived from a system originating from different cell lines is still an endogenous microsome (mixture) within the meaning of the present invention. Hybrid lysates comprising components from different families or even different species are excluded and are no part of the present invention. A hybrid lysate means that the components such as ER, Golgi, microsomes, plastids, chloroplasts and/or mitochondria are not from the same family or even not from the same species ( e. g plant, yeast, fungi, prokaryote).

A microsome according to the invention may comprise one or more of the identical TP (identical in amino acid sequence and glycosylation) or one or more different variants of the same TP as defined herein. Thus, a microsome may comprise different variants of the same TP embedded in the lipid bilayer of the same microsome. The variant may have a different glycosylation pattern and/or a different amino acid sequence. Such a microsome in an embodiment may be used in the screening according to the invention. In another embodiment the microsome is treated to achieve microsomal fragments each comprising at least one variant of the same TP embedded in the lipid bilayer. Thus, after treating the different variants of TP are isolated from each other in different microsomal fragments.

Eukaryotic CFPS can be divided into two types; those that harbour endogenous microsomal structures, and those without endogenous vesicle compartments (Thoring et al., 2019). Presently, an eukaryotic CFPS harbouring endogenous microsomal structures for post-translational modification is encompassed by the present invention. Therefore, when producing a TP it is preferred to use endogenous microsomes which enable proper TP glycosylation.

For all aspects and embodiments of the present invention, a "transmembrane protein" (TP) according to the present invention comprises at least one transmembrane domain, at least one cytoplasmic domain and at least one cytosolic domain. A TP may comprise two, three, four, five, six, seven, eight nine, ten, 11,12, 13, 14, 15, 16, 17, 18, 19, 20, 21, 22, 23, 24 or more transmembrane domains. A "TP embedded in a lipid bilayer" means that the at least one transmembrane domain of the at least one TP is orientated and embedded in the lipid bilayer of the endogenous microsome or in the lipid bilayer of a microsomal fragment derived from the endogenous microsome. TP within the meaning of the present invention encompasses type I TPs, type II TPs, type III TPs and type IV TPs with one transmembrane domain, multi-pass TPs, lipid anchored proteins, GPI-anchored membrane proteins, bitopic TPs, polytopic TPs with α-helical transmembrane domains, polytopic TPs with β-sheet transmembrane domains, peripheral membrane proteins as well as monotopic proteins. Preferably, said TP is embedded in the lipid bilayer as it would natively occur and preferably is glycosylated. Examples of TPs are listed below.

| **No** | **Family** | **Size of family** |
|---|---|---|
| 1 | GPCR #1 (Rhodopsin-like) | 5520 |
| 2 | Major facilitator | 3680 |
| 3 | ABC transporter #1 (amino acid, phosphate, ferric, nitrate, nickel, taurine, sugar) | 2469 |
| 4 | GPCR #2 (serpentine) | 1311 |
| 5 | ABC transporter #2 (multidrug resistance) | 1179 |
| 6 | Potassium channel | 1153 |
| 7 | Receptor protein kinase a | 1150 |
| 8 | Transporter #1 (cationic acid, aromatic amino acid, choline, K+ uptake) | 1082 |
| 9 | Cytochrome b #1 (N-terminal part) | 1030 |
| 10 | Cytochrome b #2 (C-terminal part) | 942 |
| 11 | ABC transporter #3 (branched amino acid, sugar, iron, zinc, manganese) | 881 |
| 12 | ATPase transporter | 754 |
| 13 | Transporter #2 (drug/metabolite, amino acid transport) | 644 |
| 14 | Transporter #3 (SecF protein export, cation multidrug efflux) | 587 |
| 15 | NADH ubiquinone oxidoreductase | 584 |
| 16 | Cytochrome P450 | 567 |
| 17 | Transporter #4 (arsenic efflux, Na+/H+ antiporter, gluconate, mannitol, Na+/sulfate symporter, Mg2+/citrate transporter) | 551 |
| 18 | Cytochrome c oxidase | 479 |
| 19 | ABC transporter #4 (heme, O-Ag, multidrug resistance, antibiotic resistance, polysaccharide export) | 471 |
| 20 | Transporter #5 (DNA damage inducible protein, virulence factor, polysaccharide export protein, Na+ driven multidrug efflux) | 468 |
| 21 | Acetylcholine receptor | 460 |
| 22 | Adenylate cyclase | 457 |
| 23 | Permease (purines, pyrimidines, sulfate) | 406 |
| 24 | Acetyl transferase | 399 |
| 25 | Glutamate receptor | 398 |

Some specific examples of potential TPs for the HTS according to the invention comprise hAQP1 (e.g. Uniprot KB: P29972, 6 TMD), Mel-hEGFR (e.g. Uniprot KB: P00533, 1 TMD), at least nine different EGFR mutations have been described, human beta-adrenergic receptor, shaker potassium channel, connexins, inositol trisphophate receptor, nicotinic acetylcholine receptor, G-protein Coupled Receptors GPCRs, ion channels, transporters, Histidine and tyrosine kinases, Cytokine receptors, Toll-like Receptors.

Within the meaning of the multiplex HTS according to the invention contacting, interaction, preferably activation and/or binding, between the same TP each provided in any number of distinct reaction zones and different analytes is screened. In another embodiment, different variants of the TP of interest are provided in any number of distinct reaction zones, the respective number of analytes are provided, contacting is performed and interaction of the same or different analytes with the different TP variants (per reaction zone) is screened. Variants of a TP comprises any genetic variants with or without resulting variants in the amino acid sequence, with or without having effect on the 3D structure and/or activity of the TP variant, single nucleotide polymorphism (SNP), deletions and/or insertion. The aforementioned is applicable to the HTS of the present invention, wherein the at least one TP is captured to a biological non-active surface or wherein the at least one TP is not captured to a biological non-active surface.

For all aspects and embodiments of the present invention a "mobile microsome or mobile microsomal fragment" comprise at least one TP embedded in the lipid bilayer of said microsome or of said microsomal fragment. A mobile microsome or mobile microsomal fragment is captured via the a least one TP to a biological non-active surface of a particle as defined herein. Provided that the at least one biologically non-active surface is on a particle, said particle carrying the captured TP remains mobile during the steps of treating, contacting, interaction and/or detection. However, the TP is not free independently from the at least one biologically non-active surface. Thus a microsome or fragment thereof is mobile because the biologically non-active surface is mobile

### Description of the Figures (Fig.)

Fig. 1: Schematic view of microsome capture and protein functional assay using receptor-ligand interaction as shown for ACE-RBD interaction in example 1. "plate" represents a biologically non-active surface according to the present invention.
Fig. 2: Microsome capture and protein structure determination using an antibody. "plate" represents a biologically non-active surface according to the present invention.

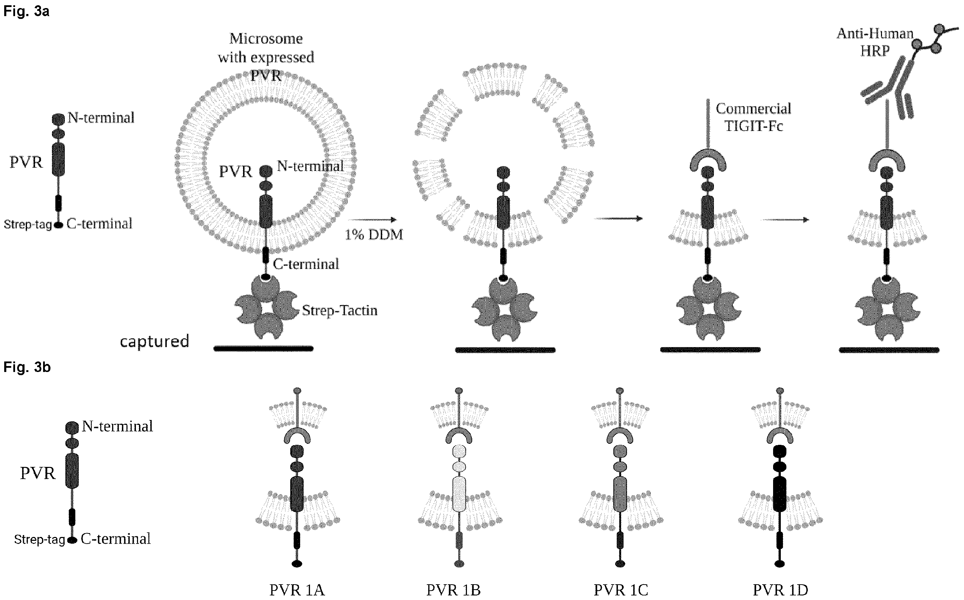

Fig. 3: **A)** Schematic view of microsome capture and protein functional assay using receptor-ligand interaction as shown for ACE-RBD interaction in example 1. "plate" represents a biologically non-active surface to which the microsome has been captured via the expressed TP according to the present invention. **B)** Functional assays using variants of the receptor. One design of a multiplex design wherein expression of variants of a particular TP is performed and different forms of a TP are tested (e.g. in parallel in a microtiter plate or another device or consumable). These versions can thereafter be analysed by high-throughput platforms such as SPR.

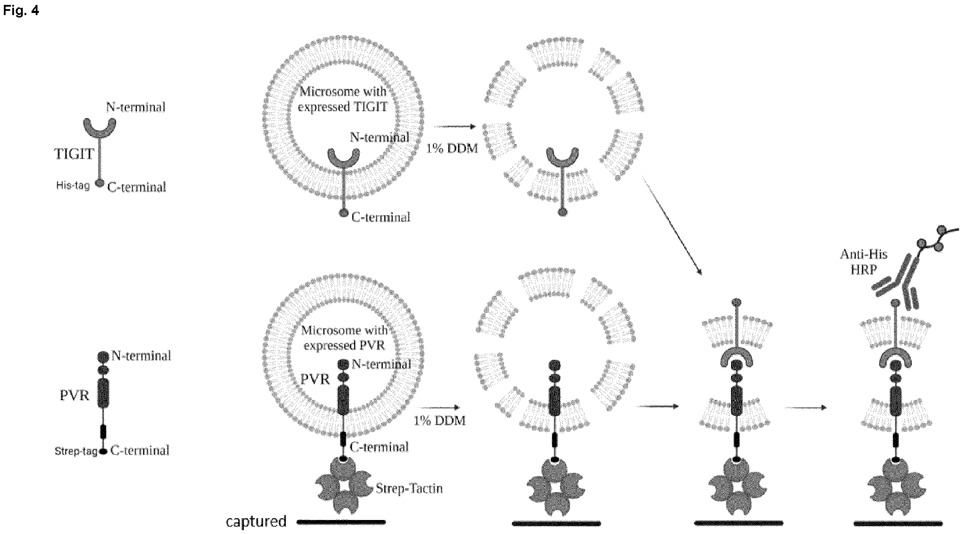

Fig. 4: Schematic view of protein-protein interaction with two different TPs expressed in two separate microsomes (preferably in two separate reaction set ups) that finally are brought together (contacting step) to study protein-protein interaction as conceptualized for PVR-TIGIT interaction (Fig. 3).
Fig. 5: Schematic view of an HTS assay designs according to the present invention, A: a screening for ligand e.g. for individualized therapy (different RBD mutants), b: screening molecules (chemical7antibody) to disrupt receptor ligand binding, C: screening ligands for orphan drugs, D: screening drug candidates
Fig. 6: Schematic view of the engineering of orientation of TP using extra transmembrane domains. The receptor domain of the TP should ideally be oriented in a way that it is inside the microsome which mimics the 'extra-cellular surface' or the oxidizing environment of the ER-Golgi. However, engineering the orientation of the receptor domain can be helpful in developing assays where the receptor and the ligand both are membrane bound proteins and could be allowed to interact before disrupting the microsomes. One possible way to engineer such orientation would be to use an extra trans-membrane domain such that an extra fold causes the receptor domain of the protein to face outside the microsome. A possible drawback of this approach could be the misfolding of the external domain due to exposure to the reducing cytosolic milieu of the lysate surrounding the microsomes. Exposure of a domain that needs disulphide bonding stabilization to the cytosolic milieu of the lysate can cause disruption of the disulphide bonds and therefore misfolding.

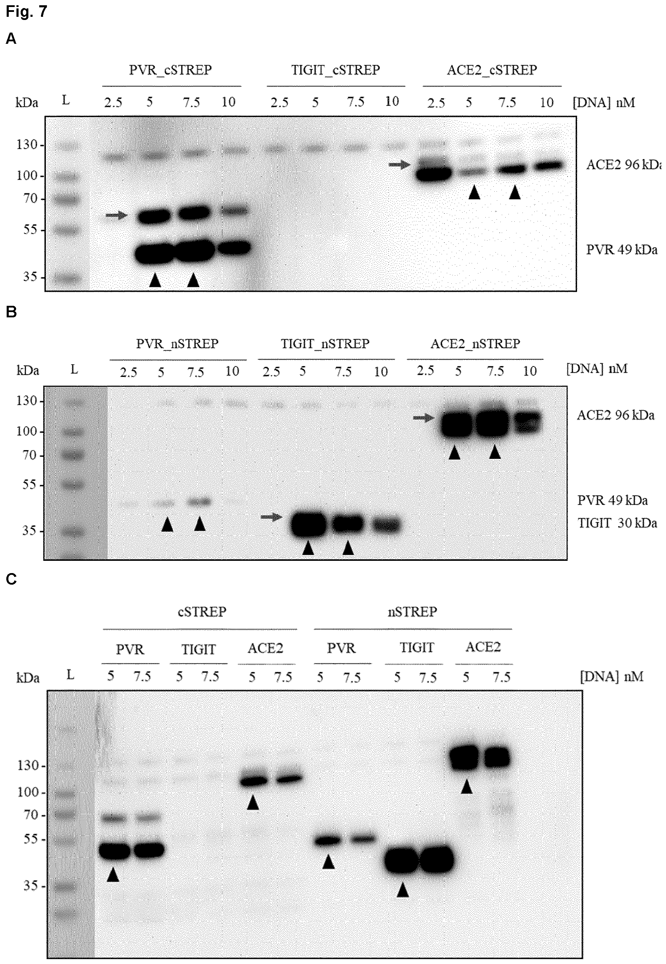

Fig. 7: Western blot analysis of ACE2, PVR and TIGIT C-terminal and N-terminal Strep-Tag^{®} II proteins expressed in ALiCE^{®}. Proteins were not purified and full lysate expressing the proteins was directly analysed on the SDS-PAGE gel. A. cSTREP tagged PVR, ACE2 and TIGIT at different DNA concentrations (2.5, 5, 7.5 and 10 nM). B. nSTREP tagged PVR, TIGIT and ACE2 at different DNA concentrations (2.5, 5, 7.7 and 10 nM). C. c and nSTREP tagged PVR, TIGIT and ACE2 at the previously chosen best DNA expressing concentrations (5 and 7.5 nM). The black arrow heads indicate the best concentrations (A and B) and the concentration chosen for following ELISA experiments (C). Red arrows indicate possible glycosylation of the expressed proteins. All proteins on the blot were probed with StrepMAB-Classic antibody conjugated with HRP. L: ladder.

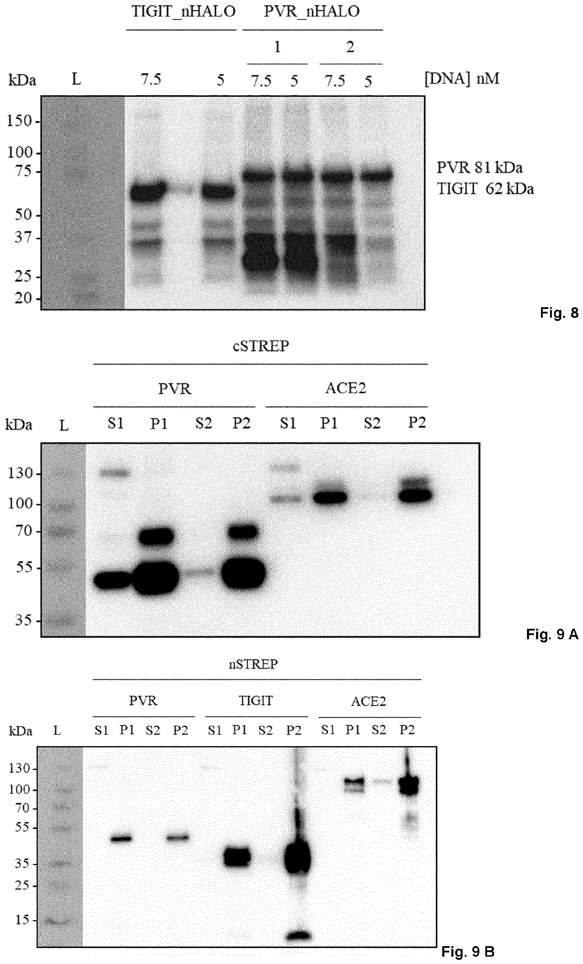

Fig. 8: Western blot analysis of TIGIT and PVR nHALO tagged expressed in ALiCE^{®}, at 5 and 7.5 nM DNA concentrations. PVR_nHALO shows duplicate bands (1 and 2) representing different colonies which correspond to different cloning events. Dual color ladder (L) was used to compare the protein band sizes. All proteins on the blot were probed with primary antibody Anti-HALO@ Tag, followed by secondary antibody Peroxidase AffiniPure Goat Anti-Mouse IgG
Fig. 9: Western blot analysis of microsomal solubilization assays for PVR, TIGIT and ACE2. A. cSTREP tagged PVR and ACE2 at different solubilization fractions. B. nSTREP tagged PVR, TIGIT, and ACE2 at different solubilization fractions. All proteins on the blot were probed with StrepMAB-Classic antibody conjugated with HRP. L: ladder; S1: first supernatant; P1: first pellet; S2: second supernatant; P2: second pellet.
Fig. 10: Indirect ELISA for PVR detection with Human CD155/PVR antibody - Goat Anti-Mouse IgG (HRP). A. ELISA graph where the Y axis represents absorbance and X axis the serial dilution factor. The more concentrated the sample is higher absorbance values are shown and vice versa. The red curve corresponds to PVR_cSTREP samples and the yellow line to PVR_nSTREP samples. B. Picture of ELISA plate where bright blue color represents reaction of detection reagent to HRP labelled antibody. More intense color is visible for cSTREP samples compared to nSTREP.
Fig. 11: Indirect ELISA for ACE2 detection and binding analysis using commercial RBD - His Tag Horseradish Peroxidase-conjugated antibody. A. ELISA graph where Y axis represents absorbance and X axis the serial dilutions. Absorbance has its highest value when the sample is concentrated and decreases with the serial dilutions. ACE2_cSTREP is represented by the red curve and ACE2_nSTREP by the yellow curve. B. Picture of the ELISA plate after addition of detection reagent, where color is visible only for ACE2_cSTREP.

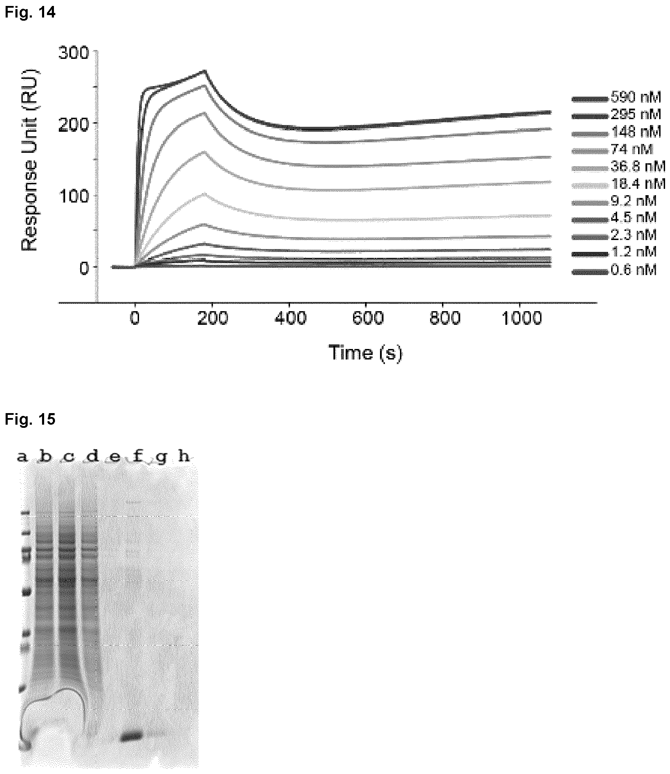

Fig. **12****:** SPR comparison of hEGF produced in either BYL or E.coli of example 2. The SDS-PAGE of hEGF purification fractions (Lanes a-h show load, wash, elution etc) is shown in Fig. 15

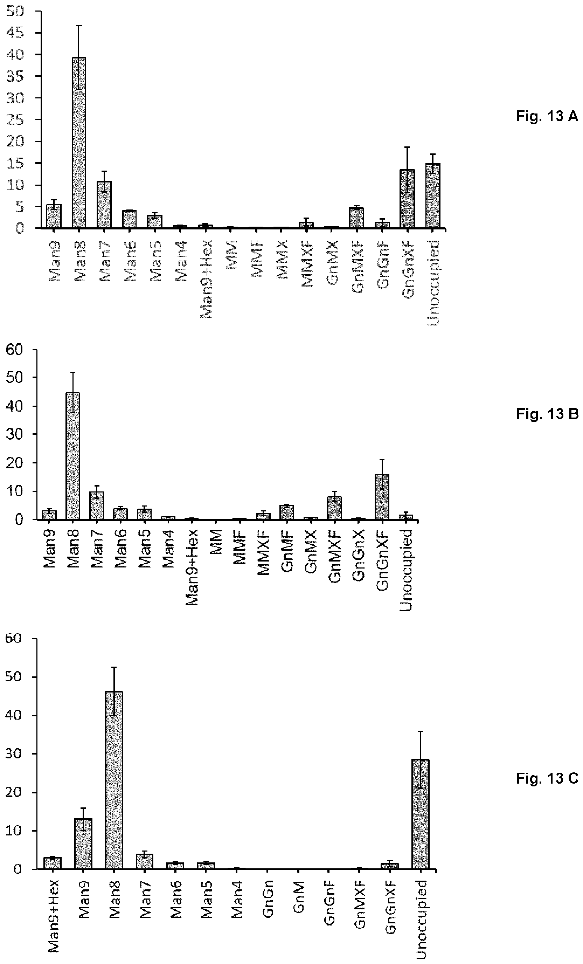

Fig. 13. Summary of LC-ESI-MS/MS analysis of N-glycan structures for RBD and adalimumab of example 4. A: RBD (SIVRFPNITNLCPFGE) with the following composition: 63,7% Oligomannose, 22,16% Hybrid (Man9+Hex, MM, MMF, MMX, MMXF, GnMX, GnMXF, GnGnF, GnGnXF) and14,86 5 Unoccupied, B: RBD (VFNATRFASVYAWNRK) with the following composition: 65,99 % Oligomannose, 32,56 % Hybrid (Man9+Hex, MM, MMF, MMXF, GnMF, GnMX, GnMXF, GnGnX, GnGnXF) and 1,43 5 Unoccupied and C: Humira with the following composition: 69,72 % Oligomannose, 1,81 % Hybrid and 28,47 % Unoccupied
Fig. 14. Binding assays of SARS-CoV-2 antigens of example 5.
Fig. **16****:** Adalimumab SPR. Immobilised antibody molecules were probed with recombinant TNFa and CD64 to show ligand and receptor binding, respectively, of example 3. A: Humira CHO TNFalpha, B: Humira CF DAK TNFalpha, C: Humira CF DB3 TNFalpha, D: Humira CF CHJ TNFalpha, E: Humira CHO CD64, F: Humira DAK CD64, G: Humira DB3 CD64, H: Humira CHJ CD64

### Examples

### Example 1

### 1. Material and methods

### 1.1 In silico cloning, plasmid design

Human DNA templates from Gateway cloning for ACE2, PVR and TIGIT proteins were first cloned in silico into the vector pALiCE02 using SnapGene^{®} software (from Insightful Science; available at www.snapgene.com). Afterwards in vitro cloning strategy was defined. HaloTag^{®}, Strep-Tag^{®} II and GST-tags were fused C- or N-terminally to the DNA templates for protein orientation testing purposes. Melittin signal peptide sequence (MSP) was N-terminally fused for microsomal targeting (Stech et al., 2014). All clones are shown in Table 1.

A template according to the present invention is encoded on a vector, preferably on a pALiCE vector. The pALiCE01 and pALiCE02 comprise a basic design (pALiCE) comprising a T7 promoter followed by the Tobacco Mosaic Virus (TMV) 5' omega leader sequence, the gene of interest (GOI, which presently encodes for the TP of interest) followed by the TMV 3' UTR. The pALiCE02 vector additionally includes the insect Melittin Signal Peptide directly before the start of the GOI which allows microsomal targeting and translocation of TP. In both plasmids, the GOI cassette is composed of an N-terminal Strep-II tag followed by a Factor Xa cleavage site and eYFP. The design of the vectors allows traditional enzyme based sub-cloning of genes into the Ncol and the Kpnl sites flanking the GOI cassette.

**Table 1: In silico designed clones with their respective tags.**

| Clones | **Tags** | **Tag description** |
|---|---|---|
| PVR_cGST_pALiCE02 | C-terminal GST tag | Made up by 211 amino acids and C-terminally fused to the protein of interest. |
| TIGIT_cGST_pALiCE02 | | |
| ACE2_cGST_pALiCE02 | | |
| PVR_nHALO_pALiCE02 | N-terminal HaloTag^{®} | Made up by 297 amino acids and N-terminally fused to the protein of interest. |
| TlGlT_nHALO_pALiCE02 | | |
| ACE2_nHALO_pALiCE02 | | |
| PVR_cSTREP_pALiCE02 | C-terminal STREP-Tag^{®} II | Synthetic peptide made of 8 amino acids. With affinity towards Strep-Tactin^{®} and N- and C-terminally fused to the protein of interest. |
| TIGIT_cSTREP_pALiCE02 | | |
| ACE2_cSTREP_pALiCE02 | | |
| PVR_nSTREP_pALiCE02 | N-terminal STREP-Tag^{®} II | |
| TIGIT_nSTREP_pALiCE02 | | |
| ACE2_nSTREP_pALiCE02 | | |

Glycosylation sites of the TPs were studied by using the online N-glycosylation prediction tool NetNGlyc - 1.0 (Gupta & Brunak, 2002), and shown schematically below:

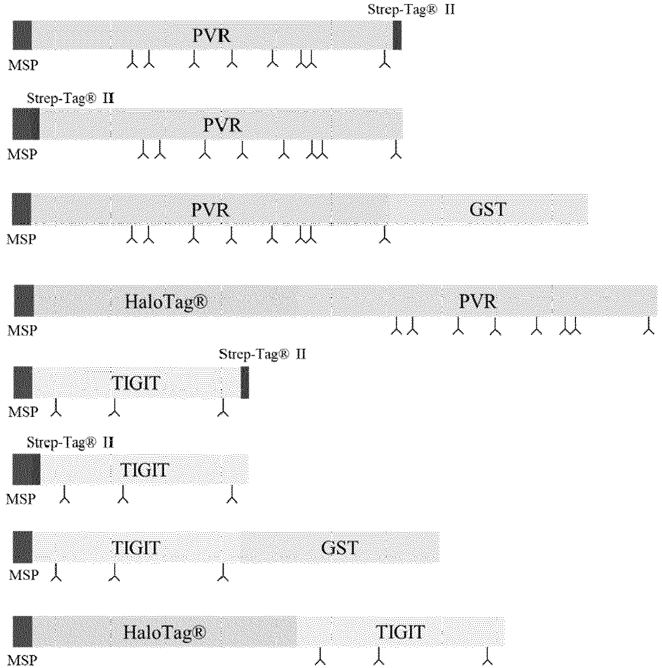
Strep-Tag® II
PVR
MSP
Strep-Tag® II
PVR
MSP
PVR
GST
MSP
HaloTag®
PVR
MSP
Strep-Tag® II
TIGIT
MSP
Strep-Tag® II
TIGIT
MSP
TIGIT
GST
MSP
HaloTag®
TIGIT
MSP

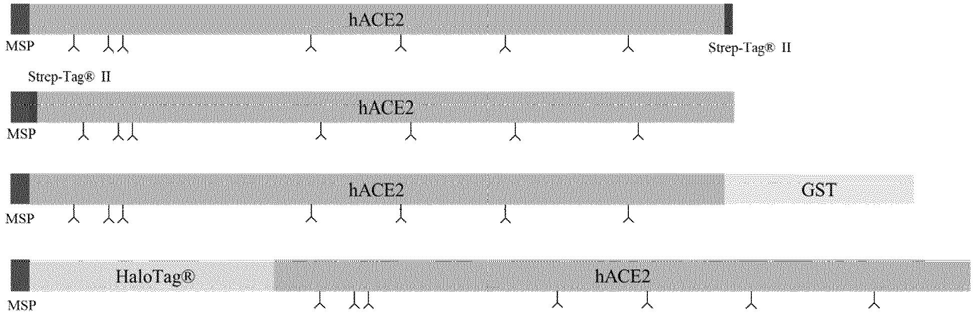
hACE2
MSP
Strep-Tag® II
Strep-Tag® II
hACE2
MSP
hACE2
GST
MSP
HaloTag®
hACE2
MSP

For the proof-of-concept PVR, TIGIT and ACE2 constructs were used with its respective tag and the signal peptide sequence (MSP) and the displayed glycosylation sites. PVR is composed of 417 amino acids has 8 N-glycans sites: 105, 120, 188, 237, 278, 307, 313 and 405. TIGIT is composed of 244 amino acids has 3 N-glycosylation sites: 53, 90 and 236. ACE2 is composed of 805 amino acids has 7 N-glycan sites: 53, 90, 103, 322, 432, 546 and 690.

### 1.2 Template amplification, cloning, plasmid extraction and sequencing

In silico primers were designed as shown below to amplify the genes of interest by PCR.

| **Name** | **Sequence** | **Vector** | **Seq-ID No** |
|---|---|---|---|
| P0001 | GTACGCACCACGTGTGATTAC | 43_pIVEX_3'UTR_Seq_R | 1 |
| P0002 | TCACATGTAATACGACTCACTATAGG | 418_pALiCE Seq Fw | 2 |
| P0750 | TAGTAATAAGGATCCTCTAGAGGTACCA AGC | VEC.FOR_cGST_nHalo_Universal | 3 |
| P0751 | CATGGCAGCCGCATAGATG | VEC.REV_cGST_nHaloUniversal | 4 |
| P0752 | CATGGCAGCCGCATAGATGTAAG | VEC.REV_cGST_nHaloUniversal_2 | 5 |
| P0753 | TCTATGCGGCTGCCATGGCTCCGTCAT CAACAAGTTTGTACAAAAAAG | FRA.FOR_cGST_Universal_v2 | 6 |
| P0754 | TCTATGCGGCTGCCATGGCTCCGTCAT CAACAAGTTTGTACAAAA | FRA.FOR_cGST_Universal | 7 |
| P0755 | CCTCTAGAGGATCCTTATTACTATTTTG GAGGATGGTCGCCAC | FRA.REV_cGST_Universal | 8 |
| P0756 | TAGTAATAAGGATCCTCTAGAGGTACCA AGC | VEC.FOR_cGST_nHalo_Universal | 9 |
| P0757 | CATGGCAGCCGCATAGATG | VEC.REV_cGST_nHalo_Universal | 10 |
| P0758 | TCTATGCGGCTGCCATGGCTATGGCAG AAATCGGTACTGGC | FRA.FOR_nHALO_Universal | 11 |
| P0759 | CCTCTAGAGGATCCTTATTACTATACCA CTTTGTACAAGAAAGCTGGG | FRA.REV_nHalo_Universal | 12 |
| P0760 | GTACCTCTAGAGGATCCTTATTACTATT TCTCGAACTGTGGATGGGACCATGCAA CCACTTTGTACAAGAAAGCTGG | FRA.REV_cStrep-Universal | 13 |
| P0761 | TATGCGGCTGCCATGGCTGCTCCGTCA TCAACAAG | FRA.FOR_cStrep-Universal | 14 |
| P0762 | TAATAAGGATCCTCTAGAGGTACCAAG C | VEC.FOR_C_Strep_Universal | 15 |
| P0763 | CATGGCAGCCGCATAGATGTAAGAAAT GTATACG | VEC.REV_cStrep-Universal | 16 |
| P0764 | AAAAGCGCTGAAAACCTGGCTCCGTCA TCAACAAGTTTG | Fragment.FOR-N_Strep_Universal | 17 |
| P0765 | CCTCTAGAGGATCCTTATTACTAAACCA CTTTGTACAAGAAAGCTGGG | Fragment.REV_N_Strep-Universal | 18 |
| P0766 | TAGTAATAAGGATCCTCTAGAGGTACCA AGC | VEC.FOR-N-strep-Universal | 19 |
| P0767 | CAGGTTTTCAGCGC I I I I I I CGAA | VEC.REV-N_Strep-Universal | 20 |
| P0768 | CTCTGCACGCTCTTTTGGACAAC | REV.GST_Seq | 21 |
| P0769 | GCATTTATGGAGTTCATCCGCC | FOR.HALO_Seq | 22 |
| P0770 | GTATACATTTCTTACATCTATGCGATGT CAAGCTCTTCCTGGCT | hACE2_cSTREP_FOR | 23 |
| P0771 | TGGATGGGACCATGCCAAAAAGGAGGT CTGAACATCA | hACE2_cSTREP_REV | 24 |
| P0772 | GCATGGTCCCATCCACAGT | pALiCE02_FOR | 25 |
| P0773 | GTATACATTTCTTACATCTATGCGATGG CCCGAGCCATGG | PVR_cSTREP_FOR | 26 |
| P0774 | TGGATGGGACCATGCCCTTGTGCCCTC TGT | PVR_cSTREP_REV | 27 |
| P0775 | GTATACATTTCTTACATCTATGCGATGC GCTGGTGTCTCCT | TIGIT_cSTREP_FOR | 28 |
| P0776 | TGGATGGGACCATGCACCAGTCTCTGT GAAGAAG | TIGIT_cSTREP_REV | 29 |
| P0777 | TGGATGGGACCATGCTTTTGGAGGATG GTCGCCAC | cGST_REV | 30 |
| P0778 | GTATACATTTCTTACATCTATGCGAATG TCAAGCTCTTCCTGGCT | hACE2_cGST_FOR | 31 |
| P0779 | GTATACATTTCTTACATCTATGCGATGG CCCGAGCCATGG | PVR_cGST_FOR | 32 |
| P0780 | GTATACATTTCTTACATCTATGCGAATG CGCTGGTGTCTCCT | TIGIT_cGST_FOR | 33 |

| | | | |
|---|---|---|---|
| Seq-ID No. 1 and 2: universal; Seq ID-No. 3 to 33: In silico designed primers | | | |

TP's genes of interest were amplified using the designed primers and PrimeSTAR GXL DNA polymerase ((1.25U/µl)*2, TaKaRa Bio) as shown in table 2, 3 and 4.

**Table 2: PCR templates with the corresponding designed primers for PCR amplification.**

| **TEMPLATE** | **Forward primer** | **Reverse primer** | **TAG** |
|---|---|---|---|
| cGST_PVR | P0760 | P0761 | C-Terminal Strep-Tag^{®} II |
| cGST_TIGIT | P0760 | P0761 | |
| cGST_ACE2 | P0760 | P0761 | |
| cGST_PVR | P0764 | P0765 | N-Terminal Strep-Tag^{®} II |
| cGST_TIGIT | P0764 | P0765 | |
| cGST_ACE2 | P0764 | P0765 | |
| cGST_PVR | P0753 | P0755 | C-Terminal GST tag |
| cGST_TIGIT | P0753 | P0755 | |
| cGST_ACE2 | P0753 | P0755 | |
| nHALO_PVR | P0758 | P0759 | N-Terminal HaloTag^{®} |
| nHALO_TIGIT | P0758 | P0759 | |
| nHALO_ACE2 | P0758 | P0759 | |
| pALiCE02 | P0762 | P0763 | C-Terminal Strep-Tag^{®} II |
| pALiCE02 | P0766 | P0767 | N-Terminal Strep-Tag^{®} II |
| pALiCE02 | P0750 | P0752 | C - Terminal GST tag and N-Terminal HaloTag^{®} |

**Table 3: PCR tube mix for amplification of the desired DNA templates and the vectors.**

| **Reactive** | **Volume** | |
|---|---|---|
| 5xPS GXL Buffer (Mg²⁺ plus)*³ | 5µl | |
| dNTP Mixture | 2µl | |
| PrimeSTAR GXL DNA polymerase (1.25U/µl)^{*2} | 0.5µl | |
| Primer Forward | 0.5µl | |
| Primer Reverse | 0.5µl | |
| DNA | 1µl | |
| MiliQ water | 16µl | |
| | **Total volume** | 25µl |

**Table 4: PCR settings used for the amplification of the inserts and vectors of interest**

| **Step** | | **Time** | **Temperature** | **Cycles** |
|---|---|---|---|---|
| 1 | Initial denaturation | 30 seconds | 98°C | 1 |
| 2 | Denaturation | 10 seconds | 98°C | 30 |
| | Annealing | 60 seconds | 60°C | |
| | Elongation | 1 minute | 72°C | |
| 3 | Final extension | 5 minutes | 72°C | 1 |
| 4 | Hold | Infinite | 4°C | ∞ |

Agarose gels (1% (w/v)) with Ethidium Bromide (0.5 µg/ml) ran for 15 minutes at 150 V to check for PCR amplicons sizes. Bands were visualized using GelDocXR+ (Bio-Rad). The marker used was 1Kb Plus DNA ladder (NewEngland Biolabs). After PCR, Gibson Assembly@ was performed. Gibson Assembly@ reaction mix (Insert: 2 µl Vector: 1 µl MiliQ water: 2 µl 2xHiFi DNA Assembly MM: 5 µl, total volume 10 µl) was prepared using NEBuilder^{®} 2xHiFi DNA Assembly Master Mix (NewEngland Biolabs). The mix was incubated at 50 °C for 30 minutes in a PCR machine. Plasmids were transferred into competent DH5α E. coli cells. Cells were incubated for 10 minutes on ice, followed by a thermo shock at 42 °C for 30 seconds. Cells were cooled down during 1 minute on ice and then incubated for 40 minutes in SOC medium (2% tryptone, 0.5% yeast extract, 10 mM NaCl, 2.5 mM KCI, 10 mM MgCI2, 10 mM MgSO4, and 20 mM glucose) at 37 °C while shacking. Eppendorf tubes were centrifuged at 3000 rpm for 1 min in a tabletop centrifuge. The supernatant was discarded and pellet was used for later plating on Agar plates containing 100 µl/ml Ampicillin (Amp). Plates were incubated at 37 °C overnight.

Colony PCR followed, amplification with specific primers as shown below in table 5 and DreamTaq Green MM (2x) (Thermo Fisher Scientific^{™}) was done. 5 colonies from each plate were chosen, swapped and introduced using a toothpick into 5 different PCR tubes containing the master mix (Content of PCR tubes for colony PCR with total volume of 10µl consisted of DreamTaq Green MM (2x): 5µl, MiliQ water: 4µl, Primer FOR: 0.5µl and Primer REV: 0.5µl) The applied PCR settings are summarized in table 6. PCR amplicon sizes were confirmed, using Agarose gels ((1% (w/v)) with Ethidium Bromide (0.5 µg/ml), as previously described.

**Table 5: Primers used for colony PCR depending on the fusion tag of each clone.**

| | **Primers** | |
|---|---|---|
| **Clone Tag** | **FORWARD** | **REVERSE** |
| STREPII tag | P0002 | P0001 |
| C-terminal GST tag | P0002 | P0768 |
| N-terminal HALO tag | P0769 | P0001 |

**Table 6: PCR settings for the colony PCR. Elongation time 1 minute per kb. ACE2 3kb long, PVR and TIGIT between 1.5-2kb.**

| **St** | **ep** | **Time** | **Temperature** | **Cycles** |
|---|---|---|---|---|
| 1 | Initial denaturation | 2 minutes | 94°C | 1 |
| 2 | Denaturation | 15 seconds | 94°C | 30 |
| | Annealing | 20 seconds | 60°C | |
| | Elongation | 2(TIGIT/PVR) -3(ACE2)minutes | 72°C | |
| 3 | Final extension | 2 minutes | 72°C | 1 |
| 4 | Hold | Infinite | 4°C | ∞ |

For sequencing NucleoSpin^{®} Plasmid purification NoLid (Macherey Nagel) was performed following the manufacturer's instructions. Purified plasmids were prepared following the sequencing provider instructions (Eurofins Genomics) and sent for sequencing. Once the sequences were confirmed, NucleoBond^{®} Xtra plasmid purification (Macherey Nagel) was done following the manufacturer's instructions to prepare plasmid for future expression in ALiCE^{®}.

### 1.3 Protein expression and western blot analysis

### Protein expression:

Protein expression was done using the cell-free system ALiCE^{®}. 96-well cell culture microplate (Greiner Bio-One^{™}) were filled with 50 µl of lysate per well to which a determined volume corresponding to different DNA concentrations was added. The DNA concentrations used were 2.5 nM, 5 nM, 7.5 nM and 10 nM. Interspaces of the wells were filled with water to prevent evaporation and maintain humidity. The plate was incubated for approximately 20 hours at 25°C with constant agitation at 500 rpm and 80% humidity in ISF1-X Climo-Shaker (KuhnerShaker).

### Semi-dry western Blot:

Lysate samples were prepared using 4x Laemmli Sample Buffer (Bio-Rad) supplemented with 10% (v/v) β-Mercaptoethanol, and incubated for 2 minutes at 98°C. Proteins were separated by electrophoresis using an SDS-PAGE gel (MiniProtean TGX Stain-Free 4-20%, Bio-Rad) that ran for 30 min at 200 V in Running buffer (250 mM Tris base, Glycine 192 mM, 1% SDS (w/v), pH 8.8). The gel was transferred to a nitrocellulose membrane using the Mini Trans-Blot^{®} Turbo Transfer System (Bio-Rad), following the manufacturer's instructions. After transfer the membrane was blocked using Blocking buffer (5% Milk (w/v) - 50mM Tris, pH 7.5, 150 mM NaCl, 0.1% (v/v) Tween-20 (TBST)) and incubated for 30 min while shacking.

The membrane was then incubated with the corresponding primary antibody StrepMAB-Classic - HRP (2-1509-001, IBA-lifesciences) 1:15.000, GST Tag Monoclonal Antibody HRP (MA4-004, Thermo Fisher Scientific^{™}) 1:2000 or Anti-HALO@ Tag (G921A, Promega) 1:10.000 diluted in Blocking buffer, for 1 hour at room temperature while shaking. After incubation 3 consecutive 5 minutes washes while shaking using TBST, were performed. A secondary antibody was used only for the N-terminal HaloTag^{®}, Peroxidase AffiniPure Goat Anti-Mouse IgG (115-035-003, Jackson ImmunoReseach) 1:10.000 diluted in Blocking buffer and incubated for 1 hour at room temperature while shaking. After incubation washes with TBST were done exactly like the previous washing step.

Immunodetection by a chemiluminescent reaction using the HRP-conjugated antibodies was done. 1:1 SuperSignal^{™} West Atto Ultimate Sensitivity Substrate (Thermo Fisher Scientific^{™}) was used for HRP-antibody detection. Protein band revelation was done using ChemStudio PLUS (Analytik Jena^{™}). PageRulerTM pre-stained protein ladder 10-250 kDa was used as a marker (Thermo FisherScientific^{™}) for N- and C-terminal Strep-Tag^{®} II samples, and Precision Plus Protein Dual Color Standards 10-250 kDa (Bio-Rad) for N-terminal HaloTag^{®} and C-terminal GST tag samples.

### 1.4 Treating of microsome in order to achieve microsomal fragments.

20 µl of the incubated lysate were pipetted inside 1.5 ml Eppendorf tubes and centrifuged at 16.000 g for 15 min. The supernatant or S1 was collected in a different tube, while the pellet or P1 (containing microsomes) was resuspended in 1% Dodecacyl-b-Maltoside (DDM) diluted in PBS (137 mM NaCl, 2.7 mM KCI, Na2HPO4, 1.8 mM KH2PO4) and incubated on ice for 1 hour. P1 was centrifuged at 16.000 g for 15 min. From this centrifugation two fractions were collected: the supernatant or S2 (containing solubilized microsomal proteins) and the pellet or P2 (containing microsomal residues). P2 was resuspended thoroughly in PBS. Once all the products were ready (S1, P1, S2, P2) the protein samples were prepared for western blot as described above. A schematic overview of the experimental procedure is shown below:

S1
Analysis
Aliquot on Gel
Centrifugation
S1
P1
Lysate
(Post-Reaction)
S2
Protein
purification
Resuspension
1% DDM
(Incubation
ice 1 hour)
P1
P1
Centrifugation
S2
P2
Analysis
Aliquot on Gel
Analysis
Aliquot on Gel
P2

### 1.5 Capturing of microsomes capture and protein binding assays.

ACE2 and PVR expressing lysate were used for indirect ELISA. experimental set up is shown in Figures 1 to 4.

### INDIRECT ELISA

### Microsome capturing:

Lysate samples were centrifuged at 16.000 g for 15 min to concentrate microsomes, the pellet was resuspended in PBS. Concentrated microsome samples were added directly to each well of the Strep-Tactin^{®} XT coated microplates (1-4101-001, Iba), samples were prepared in triplicates. A series of unknown dilutions starting from the concentrated sample (1) were performed until reaching 0 (Starting with concentrated sample 1 and performing 8 dilution steps 1:100: 0,5->0,25 ->0,125->0,0625->0,03125->0,015625->0,0078125-> discard). The plate was incubated at room temperature for 1 hour.

### Protein binding:

1% DDM in PBS was added to each well, and the plate was incubated for 15 minutes at room temperature. The plate was then washed 3 times with PBS - 0.05% Tween (v/v) (PBST). The wells were blocked adding 1x Blocking buffer (ab126587, Abcam) to prevent unspecific binding, and incubated for 1 hour at room temperature. A washing step like the previous one was performed.

For PVR microsome capturing detection, Human CD155/PVR antibody (MAB25301, Bio-Techne) 1:5000 in Blocking buffer was added. For PVR protein binding to TIGIT, Recombinant Human TIGIT Fc Chimera (7898-TG, Bio-Techne) in Blocking buffer at 2.5 µg/ml was added. For ACE2 microsome capturing and protein binding analysis, commercially available RBD SARS-CoV Spike/RBD Protein (RBD, His Tag) (40150-V08B2, SinoBiological) and purified RBD SARS-CoV-2 were diluted to a concentration of 2.5 µg/ml in Blocking buffer. Plates were incubated at room temperature for 1 hour and followed by a washing step

For protein detection, a detection antibody (HRP labelled) that binds to the protein was added. For PVR's structural antibody detection Peroxidase AffiniPure Goat Anti-Mouse IgG (115-035-003, Jackson ImmunoResearch) 1:10000 in Blocking buffer was added. For PVR-TIGIT biding detection Goat anti-Human IgG F (ab') 2:HRP (0500-0099) 1:10000 in Blocking buffer was added. For ACE2-commercial RBD/purified RBD binding detection His Tag Horseradish Peroxidase-conjugated antibody (MAB050H, Bio-Techne) 1:4000 and StrepMAB-Classic - HRP (2-1509-001, IBA-lifesciences) 1:15.000 in Blocking buffer were added. Plates were incubated at room temperature for 1 hour and then washed.

For protein visualization, Detection regent TMB ELISA substrate (High Sensitivity) (Abcam) was added to each of the wells and incubated at 37 °C in the dark for 15 minutes. Once the wells had developed some color absorbance was measured (650nm) using the TECAN Infinite M1000 Pro machine and Tecan i-Control 2.0 software with the settings shown in table 8. Absorbance values were analyzed using Excel to build absorbance/dilution graphs.

**Table 8: Lecture settings for Tecan i-Control 2.0 Software.**

| **Parameter** | **Settings** | |
|---|---|---|
| Shaking | Duration | 10 seconds |
| | Mode | Orbital |
| | Amplitude | 2 mm |
| | Frequency | 306 rpm |
| Absorbance | Wavelength | 650nm |
| | Flashes | Number of flashes: 25 |
| | | Settle time: 10ms |

### 2. Results

### 2.1. Successful expression of membrane proteins in ALiCE^{®}.

To express the TP of interest, in vitro cloning was performed in pALiCE02 vector as shown in table 9. The generated plasmids were used to express the proteins in ALiCE^{®} at different plasmid concentrations, and expression was checked by western blot analysis.

**Table 9: Clones with insert successfully cloned in the vector pALiCE02.**

| N° | Insert | Clone name | Plasmid name |
|---|---|---|---|
| 1 | cGST-PVR | PVR_cGST_pALiCE02 | |
| 2 | cGST-TIGIT | TIGIT_cGST_pALiCE02 | |
| 3 | cGST-ACE2 | ACE2_cGST_pALiCE02 | |
| 4 | nHALO-PVR | PVR_nHALO_pALiCE02 | pLB0339_pA02_PVR_nHALO |
| 5 | nHALO-TIGIT | TIGIT_nHALO_pALiCE02 | pLB0340_pA02_TIGIT_nHALO |
| 6 | nHALO-AC E2 | | |
| 7 | cSTREP-PVR | PVR_cSTREP_pALiCE02 | pLB0344_pA02_PVR_cSTREP |
| 8 | cSTREP-TIGIT | TIGIT_cSTREP_pALiCE02 | |
| 9 | cSTREP-ACE2 | ACE2_cSTREP_pALiCE02 | pLB0345_pA02_hACE2_cSTREP |
| 10 | nSTREP-PVR | PVR_nSTREP_pALiCE02 | pLB0341_pA02_PVR_nSTREP |
| 11 | nSTREP-TIGIT | TIGIT_nSTREP_pALiCE02 | pLB0342_pA02_TIGIT_nSTREP |
| 12 | nSTREP-ACE2 | ACE2_nSTREP_pALiCE02 | pLB0343_pA02_hACE2_nSTREP |

C-terminal Strep-Tagged proteins showed bands for PVR_cSTREP and ACE2_cSTREP however, no bands were visible for TIGIT_cSTREP (Fig. 7A). PVR_cSTREP had two visible bands one at the expected size (49 kDa) and another band running higher which, might be a sign of glycosylation. PVR_cSTREP showed bands for all DNA concentrations except 2.5 nM. Moreover, the highest concentration showed less band intensity which could indicate saturation of the system at higher DNA concentrations than 7.5 nM. ACE2_cSTREP had visible bands for all DNA concentrations at the expected size (96 kDa). A higher band that could correspond to the glycosylated protein was visible for all ACE2_cSTREP concentrations. For TIGIT_cSTREP no bands were visible. Thus, the presence of bands on the blot is indicative of protein expression. In each sample a non-specific band at 120 kDa was observed.

For N-terminal Strep-Tagged proteins, bands corresponding to its expected size were visible for PVR_nSTREP (49 kDa), TIGIT_nSTREP (30 kDa) and ACE2_nSTREP (96 kDa) (Fig. 7B), which meant successful protein expression. PVR_nSTREP bands were visible at all concentrations but with low intensity. Besides, PVR_nSTREP did not show any signs of glycosylation and only one band was visible on the blot. ACE2_ nSTREP showed bands at all concentrations except 2.5 nM. ACE2_nSTREP bands ran higher than expected and two bands were identified at 5, 7.5 and 10 nM DNA concentrations which could be due to glycosylation. Finally, TIGIT_nSTREP showed bands for all concentrations except 2.5 nM running higher than expected, a sign of possible glycosylation.

A blot to test the reproducibility of the experiment with the two best DNA concentrations previously analyzed in blots for cSTREP and nSTREP samples was done (Fig. 7C). Differences between band intensity for C- and N-terminal fusion was observed for each construct. PVR_cSTREP showed stronger band intensity and two bands, possible result of glycosylation, while PVR_nSTREP was less intense and there was only one band present. TIGIT _cSTREP showed no bands while TIGIT_nSTREP showed a very intense higher than expected band probably due to glycosylation. Finally, the ACE2_cSTREP band intensity was lower than for ACE2_nSTREP where an intense and higher than expected band was visible. From this blot the best concentration, 5 nM, was chosen and used for protein solubilization and ELISA experiments. Western blot was also used to check N-terminal HaloTag^{®} protein expression for PVR and TIGIT. Bands on the expected size for PVR (81 kDa) and TIGIT (62 kDa) were visualized on the blot (Fig. 8). However, higher levels of degradation for these proteins were visible. For PVR two different clones were used for expression analysis on the blot, both of which showed similar results. Protein expression analysis indicated that PVR_c/nSTREP, TIGIT_nSTREP, ACE2_c/nSTREP, PVR_nHALO and TIGIT_nHALO were successfully expressed in ALiCE^{®}. While GST tagged constructs and TIGIT_cSTREP were not expressed. The results showed that Strep-Tag^{®} II protein constructs worked best for protein expression. Hence, Strep-Tag^{®} II constructs were chosen to continue with the following experiments

### 2.2 DDM is unable to dissociate MPs from the microsomal membrane

To find out if the microsomal membrane could be disrupted and if the TPs could be solubilized using a mild detergent (1% DDM), microsomal solubilization analysis took place as described above. Expression of the lysate and analysis of the centrifuged fractions after DDM addition was done by western blot. DNA concentrations used were determined by previous western blot analysis (Fig. 7C).

A blot analysis for microsomal solubilization assays for C-terminal Strep-Tag^{®} II PVR and ACE2 proteins was done (Fig. 9 A). All fractions showed bands in the expected sizes but the intensities differed between fractions. As expected higher band intensity was visible for P1 and P2 fractions which correspond to microsomal membrane residues. However, the proteins could also be detected in the S1 fraction with higher intensity for PVR_cSTREP samples. Only one band for S1 PVR_cSTREP was visible which meant that even though proteins were present it corresponded to non-glycosylated proteins. The S2 fraction showed a vague band but not strong enough to assume solubility of these proteins.

N-terminal Strep-tag^{®} II PVR, TIGIT and ACE2 were analyzed in another blot (Fig. 9B). For all samples bands on the expected sizes were only visible on P1 and P2 fractions. These fractions correspond to the microsomal membrane residues. ACE2_nSTREP showed a low intensity band for S2 not strong enough to assume solubility of the protein.

TPs were not solubilized when using 1%DDM (Fig. 8) and remained in the P1 and P2 fractions where microsomal membrane residues are expected. It is possible that a mild detergent like DDM is unable to dissociate the membrane proteins from the microsomal membranes and the membrane bound MP still ends up in the pellet fractions. Stronger detergents may be needed to solubilize TPs.

### 2.3 Correctly folded microsomal PVR can be captured for HTS.

Indirect ELISA experiments were done to capture microsomes expressing PVR and find out the protein's orientation. Furthermore, protein structure and binding to its ligand (TIGIT) were studied as described above. PVR capturing was done by using the C-terminal Strep-Tag^{®} II. Structural antibodies and commercial TIGIT were later used to check the protein structure and binding activity.

By measuring the absorbance a graph where absorbance together with the corresponding dilution factor was built (Fig. 10A). The more concentrated the sample was the higher the absorbance values. Thus, the more concentrated the lysate was more microsomes expressing the proteins were captured in the plates and subsequently more proteins could bind to the structural antibody giving blue coloration. PVR_cSTREP absorbance values were higher than PVR_nSTREP. Samples were visualized by the addition of a detection reagent to the wells which will give a blue coloration (Fig. 10B). Color was visible for both samples however, PVR_cSTREP samples showed stronger color than PVR_nSTREP samples. Therefore, C-terminally tagged proteins were captured on the plates at higher concentrations than N-terminally tagged proteins. For PVR_cSTREP the second line of wells showed a difference in coloration compared to first and third line. Which was a possible result of an error of the multichannel pipette. Further testing for protein binding was done using commercially available TIGIT, but the samples showed no color. PVR and TIGIT were not binding.

Indirect ELISA experiments showed that the microsomes expressing PVR were captured in the ready to use Strep-Tactin^{®} ELISA plates (Fig. 10). Furthermore, the proteins were oriented as predicted with C-terminus outside the microsome, since the samples for cSTREP tagged proteins showed more coloration (Fig. 10B) and higher absorbance values (Fig. 10A) than those for nSTREP tagged proteins. PVR was correctly folded showing color when binding to its structural antibody, but it was unable to bind to commercial TIGIT. Experiments could not be statistically analyzed due to the lack of a standard curve however, qualitative analysis was performed. Future experiments need to be carried out to compile more data and to develop a standard curve to quantify results. Moreover, analysis of the binding between PVR and TIGIT should be done.

### 2.4 Correctly folded microsomal ACE2 can be captured for HTS, binding effectively to RBD.

Indirect ELISA experiments were used to capture microsomes expressing ACE2 and study the binding of the protein to its ligand (RBD). ACE2 proteins were captured using their C-terminal Strep-Tag^{®} II. Commercial RBD (His tagged) and purified RBD together with corresponding detection antibodies were used to determine the protein's structure and binding efficiency.

A graph representing absorbance and the dilution factors of samples was built by measuring sample absorbance (Fig. 11 A). For ACE_cSTREP the more concentrated the sample was the higher the absorbance measurement therefore, more proteins were captured. ACE_nSTREP showed very low close to 0 values for all the dilutions. Absorbance values were higher for ACE2_cSTREP than for ACE2_nSTREP. Higher concentrations of C-terminally tagged proteins were captured on the plates compared to N-terminally tagged samples. ELISA plates were visualized by the addition of a detection reagent to the samples (Fig. 11 B). Brighter color could be clearly observed for ACE_nSTREP, while ACE_nSTREP showed barely/no blue coloration. This proved that microsomes expressing C-terminally tagged proteins were captured. Furthermore, coloration was the sign of effective binding between ACE2 and RBD.

Purified RBD did not show coloration when experimental studies were developed. Purified RBD was produced in the laboratory and continuous unfreezing of the sample could result in a non-active protein and therefore, be the reason of failure.

Indirect ELISA experiments using ready to use Strep-Tactin^{®} plates proved that ACE2 was as expected synthesized with C-terminus outside the microsome allowing microsome capturing (Fig. 11). ACE2 proteins were oriented with the C-terminus outside the microsome and N-terminus inside. Moreover, addition of commercially available His-tagged RBD followed by detection with anti-His antibody revealed that ACE2 was properly folded and bounded effectively to its ligand. Experiments could not undergo statistical analysis due to the lack of a standard curve. All results provided correspond to qualitative data analysis obtained from the experiments.

### 3. Discussion

The results of the proof-of-concept experiment shows that successful expression of the selected TPs such as PVR, TIGIT and ACE2 in the CF system ALiCE^{®} is possible, that is derived from BY-2. Moreover, microsomes expressing C-terminal Strep-tag^{®} II PVR and ACE2 were captured on ELISA plates. Microsome capturing using TPs is a novel experimental design that allowed the study of these TPs and further development of the basis of High Throughput drug-target Screening platforms. ACE2 was expressed, correctly folded and showed effective binding to its ligand RBD of SARS-CoV-1.

To express TPs of interest in ALiCE^{®}, it was shown that it is feasible by cloning the genes of interest into the pALiCE02 vector. Four different templates were designed for each TP where two C-terminal and two N-terminal tags were fused. Tags are usually used to improve protein production or confer new properties for characterization of the target proteins. Presently tags were also used to capture the microsomes and to study the protein's orientation. Strep-Tag^{®} II constructs showed good expression. None of the GST tagged constructs showed expression, even though GST is a frequently used in protein expression, affinity and solubility assays and is the most common fusion tag for pull-down assays. One hypothesis that could explain failure of expression of GST-tagged proteins could be related to the nature of the GST protein. GST tends to dimerize, which could lead to aggregation of certain target proteins, specially oligomeric proteins. Therefore, GST is a poor choice for tagging oligomeric proteins. PVR, TIGIT and ACE2 are oligomeric proteins and when fused to GST they could form large complexes or aggregates that would hamper protein expression.

The proof-of-concept experiment showed that the present invention overcomes the biggest challenges of producing TPs that is integrated into a lipid bilayer and synthesized with the correctly folded structure. In ALiCE^{®}, which is the preferred BY-2 lysate according to the present invention, endogenous microsomes originate from the ER and Golgi. Presently, to target TPs to the microsomes, the Melittin signal peptide (MSP) was fused to each construct. MSP is able to target TPs to the microsomes (Stech et al., 2014). Presently TPs were effectively targeted towards microsomes and embedded in their membranes, integrating TPs into a lipid bilayer. However, such a MSP is not obligatory and it depends on the TP of interest whether a MSP is necessary for capturing or not. Moreover, ELISA experiments proved that in line with the hypothesis, TPs were synthesized with the C-terminus outside the microsomal membrane and N-terminus inside the microsome. This has allowed the development of a novel technique where microsomes could be captured using C-terminally fused tags and to develop binding and competition assays.

In particular for therapeutic proteins, which may be identified herein as a ligand and therefore as a drug candidate, post-translational modifications (PTMs) such as glycosylation play a crucial role in protein-protein interaction. Achieving glycosylation that enables functional proteins is another challenge for protein production. Presently glycosylation for the glycoproteins PVR, TIGIT and ACE2 was tested. Since ALiCE^{®} is a CFPS system derived from BY-2 Nicotiana tabacum cells, PTMs may differ from TPs native state. Expression analysis in ALiCE^{®} showed signs of possible glycosylation based on the differences on the expected protein sizes. In the case of ACE2 in both the c/nSTREP constructs, TIGIT_cSTREP and PVR_cSTREP constructs. Moreover, ELISA studies proved that proteins were correctly folded, which indicates undergoing of correct PTMs because they could bind to either its ligand (ACE2-RBD) or to the structural antibody (PVR-antiCD155). The presence of glycans is tested by the treatment of the proteins of interest with PNGase F, which indicates the presence of N-glycans. Western blot analysis (Fig. 7 to 11) confirmed possible PTMs, like glycosylation, on the expressed proteins. Thus, the present invention produces effective, properly folded proteins without any additional step in order to achieve glycosylation of the TPs at it would be necessary in a prokaryotic system (ECE).

Drug development and biochemical and structural studies of TPs require the expression of large amounts of proteins. In vivo systems have so far been the preferred systems for protein production. Chinese Hamster Ovary (CHO) cells are the most popular and standardized eukaryotic cell line for protein production (Thoring et al., 2016). However, CHO cells have some limitations when it comes to TP production. TPs are proteins difficult to express in living cells and production of large amounts of these proteins will cause deleterious effects on cell growth and, eventually, death. Many different approaches to overcome the limitations have been tested on in vivo systems which, eventually would make it more time consuming and expensive. Presently, it has been shown for the first time, that a BY-2 lysate (commercially available as ALiCE^{®}) is effective and can replace in vivo systems for the expression of the TPs of interest without running into the in vivo system's limitations. Moreover, ALiCE^{®} proved to be a fast tool for TP production where the plasmid of interest and lysate are the only requirements for experimental set up.

Treating microsomes with a mild detergent proved that membrane proteins were not dissociated from the lipid bilayer of the microsome. Therefore, solubilization of TPs was not possible and stronger detergents should be used. Currently, the most used techniques for TP solubilization involve the use of detergents (Salipro Biotech AB, 2018) yet, there are many disadvantages associated to them. For instance, the use of detergents is related with protein instability and poor applicability in structural and biophysical studies (Salipro Biotech AB, 2019). Another technique that has been used for over 15 years are nanodiscs. Nanodiscs provide a lipid environment but do not bind tightly to the transmembrane protein which limits their application. A new technique called Salipro^{®} technology allows the reconstitution of the purified TP into a lipid environment and has recently been used for protein purification. ALiCE^{®} is a good TP production system but, purification of TPs could not be achieved using mild detergents. It was proven that TPs are embedded stably within the lipid bilayer of the microsome according to the present invention. Using the inventive production system, preferably ALiCE^{®}, as an expression system for TP and combining it with the Salipro^{®} technology could become the future of TP characterization.

Until recently, CFPS systems were not a part of the drug discovery pipeline because there were many problems associated with this technology. However, this will change with the present invention that provides an optimized and standardized system for a high throughput screening (HTS) for ligands of TP embedded in a lipid bilayer. The present results show the potential of the inventive HTS as a drug target platform based on TPs. Expression of the TPs, microsome capturing and interaction (binding) assays according to the present invention allows to set up the principles of a HTS drug screening platform based on TPs in ALiCE^{®}. Competition assays using promising compounds, like the monoclonal antibody (mAb) CR3022 (Tian et al., 2020) and chemicals like corilagin (Yang et al., 2021), that efficiently hampered the binding between ACE2-RBD will be the next steps to develop HTS. Moreover, lithyronine (Zhou et al., 2020), which was proven to hamper the binding between TIGIT-PVR, could act as control for PVR-TIGIT assay and help find new potential compounds. The combination of TP production in the CFPS system, ALiCE^{®}, together with the development of binding and competition assays will become the basis of HTS platforms. These platforms could change the pharmaceutical industry and speed up the whole drug discovery process. In conclusion presently it has been shown that the use of the CF system ALiCE^{®} is an easy and fast system replacing prior art systems for TP production.

### Example 2

### Functional expression of a complex peptide, human epidermal growth factor (hEGF)

Human epidermal growth factor (hEGF) has been extensively investigated for its potential ability to promote rapid healing of serious injuries, such as cuts, burns, and diabetic ulcers. Although hEGF promises potential clinical value, the growth factor is restricted to the treatment of chronic diabetic ulcers because of its high production cost and poor stability. Mature hEGF is a complex peptide of 53 amino acids that possess three intramolecular disulphide bonds and was chosen as another model protein with one transmembrane domain to challenge the capabilities of the BY-2 system as an expression host for bioactive hEGF.

For detection of interaction, presently binding, of the hEGF with Surface Plasmon Resonance (SPR) as a spectroscopic assay was used.

The hEGF gene with an N-terminal Strep-II tag was cloned into pALiCE01 and pALiCE02 to evaluate both cytosolic and microsomal expression (Fig. 15). Microsomal hEGF expression was observed but not cytosolic, indicating that microsomal targeting is mandatory for hEGF production from ALiCE^{®} . Single-step streptavidin affinity purification was sufficient to extract soluble hEGF at high purity (Fig. 15). Binding of the purified protein to the cognate epidermal growth factor receptor (EGFR) was used as a surrogate measure of bioactivity and analyzed by SPR (Fig. 12). hEGF expressed in the BYL showed comparable or improved binding kinetics compared to a commercial hEGF reference product expressed from E. coli, suggesting correct folding and disulphide bond formation. Prokaryotic CFPS systems require extensive engineering to enable disulphide bond formation that can be circumvented by the use of BYL microsomes.

### Example 3

### Folding, disulphide bonding, and activity of complex multi-subunit proteins targeted to the microsome

We have shown that BYL can produce functional eYFP and GOx at various scales and that functional proteins as diverse as multi-pass transmembrane GPCRs and small, soluble peptides are also possible. Protein function is intrinsically linked to correct folding but further molecular characterisation is warranted, to prove that the microsomes of this eukaryotic CFPS system are able to produce recombinant proteins at the same quality as a cell and with batch-to-batch consistency.

Thus, a varied panel of model proteins were selected for further study of folding and post-translational modification capabilities, namely: the enzyme GOx, a homodimer comprised of 80kDa monomers that are covalently linked by disulphide bond and each possessed of 8 N-glycosylation sites; the receptor binding domain (RBD) of the spike protein from the SARS-CoV-2 virus, a monomer of 52kDa with four intramolecular disulphide bonds and two N-glycosylation sites; and the therapeutic monoclonal antibody adalimumab (Humira^{®}), a complex heterotetramer of 150kDa comprised of two heavy and two light chains, together containing two N-glycosylation sites, twelve intramolecular and four intermolecular disulphide bonds.

Adalimumab heavy and light chain genes were cloned directly into pALiCE02 vectors for microsomal targeting whilst RBD and GOx genes were prepared similarly with additional N-terminal Strep-II tags. Proteins were expressed in 5mL reactions using triplicate, independent batches of BYL and the resulting microsomes were processed by dodecylmaltoside detergent treatment for protein release and subsequent Protein A or streptavidin affinity purification. For adalimumab, a 1:1 ratio of heavy and light chain pALiCE02 plasmids was sufficient to produce full-length IgG molecules at the expected 150kDa ratio. Various assemblies of heavy and light chain were also visible in the non-reducing SDS-PAGE gel but analytical size-exclusion chromatography resolved a single major peak for all three batches. The functional binding activity of these samples towards the TNFα ligand and the Fc-gamma receptor CD64 was assessed by SPR in a comparison with a commercial adalimumab reference product expressed from CHO cells (BYL-mAb and CHO-mAb, respectively; Fig. 15).

### Assay Set-Up:

CM5 S-Series Chip Biacore/Cytiva) functionalized with recombinant Protein A (P7837/LotNumber SLBT1697, Sigma) using EDC/NHS Coupling Kit (Biacore/Cytiva BR-1000-50). Conditions: Temperature 25°C, 30µl/min Flowrate, Buffer: HBS-EP, pH7,4

### Reagents:

huCD64/human FcRI, (R&D Systems 1257FC/LotNumber MOM2119041, 50kD, Reference IgG Adalinumab (Humira) (ABIN5668145), Human TNFa , Lenio derived Humira (Alice) 3 Batches

### Kinetic Run:

Serial dilutions of the ligands (CD64 and TNFa) starting at 30nM CD64 and 60nM TNFa
Capture of antibodies around 170RU
On rate 180s, Off rate 420s
2 Buffer Blanks distributed over each series.

### Results:

For TNFα binding, BYL-mAb demonstrated slightly stronger affinity than CHO-mAb, with an average KD (M) value of 1.34x10-10 compared to 1.85x10-10. Conversely, BYL-mAb showed weaker binding to the CD64 receptor than CHO-mAb (3.53x10-10 compared to 1.58x10-10). Considering that the adalimumab mechanism of action is the neutralisation of free TNFα, this enhanced ligand binding and weaker receptor binding may combine for an improved therapeutic effect when this monoclonal antibody is produced in BYL compared to mammalian cell systems. The data are summarized below and depicted in Fig. 15:

| Fig. | Assay | ka (1/Ms) | kd (1/s) | KD(M) |
|---|---|---|---|---|
| A | Humira CHO TNFalpha | 4.53E+05 | 8.37E-05 | 1.85E-10 |
| B | Humira CF DAK TNFalpha | 4.07E+05 | 5.01E-05 | 1.23E-10 |
| C | Humira CF DB3 TNFalpha | 4.78E+05 | 5.83E-05 | 1.22E-10 |
| D | Humira CF CHJ TNFalpha | 4.00E+05 | 6.24E-05 | 1.56E-10 |
| E | Humira CHO CD64 | 9.59E+05 | 1.52E-04 | 1.58E-10 |
| F | Humira DAK CD64 | 7.87E+05 | 2.72E-04 | 3.46E-10 |
| G | Humira DB3 CD64 | 6.86E+05 | 2.52E-04 | 3.67E-10 |
| H | Humira CHJ CD64 | 7.58E+05 | 2.63E-04 | 3.46E-10 |

| | **CHO** | **CF-DAK** | **CF-DB3** | **CF-CHGJ** |
|---|---|---|---|---|
| CD64 | 162/43 | 185/42 | 436/93 | 185/44 |
| | 0,29/0,265 | 0,29/0,227 | 0,29/0,21 | 0,29/0,237 |
| | 91%/- | 78%/85% | 72%/79% | 81%/89% |
| TNF-alpha | 162/52 | 185/62 | 244/73 | 185/63 |
| | 0,32/100% | 0,33/103% | 0,3/93% | 0,34/106% |

### Example 4

### N-linked glycosylation of glycoproteins expressed in ALiCE^{®}

Previous reports of glycoproteins produced in eukaryotic CFPS have demonstrated the presence of N-glycosylation using simple SDS-PAGE mobility shifts after PNGase F-mediated deglycosylation. However, there has been no detailed analysis on the composition of these N-glycans has been provided as the requisite sample amounts for mass spectrometric characterisation were unobtainable. Successful scaling of the BYL system has enabled sufficient sample for this more in-depth examination and so we analysed the N-glycan structures of the three GOx, RBD and adalimumab preparations that were also used for disulphide bond characterisation.

PNGase F cleavage of N-glycans was followed by liquid chromatography electrospray ionization tandem mass spectrometric identification and assignment of structures (LC-ESI-MS/MS; Fig. 13) The study revealed a high degree of N-glycosylation occupancy and reproducibility across batches, regardless of the protein model. The N-glycan profile is broadly consistent across all N-glycosites and demonstrates a dominant Man8 species. Similarly to recombinant proteins produced in whole Nicotiana spp. plants, plant-specific glycosylation features of α1,3-fucosylation and β1,2-xylosylation were also observed at some N-glycosites, especially within the RBD protein. One glycosylation position of the GOx protein was not detected and, therefore, not characterized. This site was located within a weakly charged peptide of approximately 50 amino acids, presumed to have hindered detection within the mass range of the MS instrument.

### Example 5

### Bioactive expression of a vaccine subunit candidate, SARS-CoV-2 spike protein receptor-binding domain

Herein we have shown the BYL system to be capable of producing recombinant SARS-CoV-2 spike protein RBD within 48 hours, with suitable disulphide bonding and with consistent batch-to-batch N-glycosylation. This protein is considered an important target for developing a SARS subunit vaccine. Indeed, the majority of the potent neutralising antibodies against SARS-CoV-2 target the RBD (REF). Reports also suggest that the full S1 subunit of the spike may be a more potent vaccine as it induces better IgG and IgA antibody levels compared to RBD alone (REF). To challenge this concept, we expressed both RBD and this S1 subunit from the pALiCE02 vector and with different HaloTag and Strep-II tag conformations.

Initial confirmation of functional RBD binding to the human receptor angiotensin-converting enzyme 2 (hACE2) was obtained for N-terminal Strep-II-tagged RBD using SPR (Fig. ). The analysis revealed the RBD produced in ALiCE to bind to immobilized hACE2 receptor with a higher affinity (Kd= 32 nM) than RBD protein produced in mammalian and human cells (Fig. ). Antigen reactivity to two commercial antibodies was then assessed, chosen based on the recognition of either linear of conformational epitopes. We observed similar reactivity of both antibodies against recombinantly expressed RBD and S1 from both BYL CFPS and HEK293 cells. Importantly, the reactivity against S1 was weaker than that for RBD. Finally, to demonstrate true biological relevance of proteins produced in BYL, we found clear serological reactivity of the antigen samples (Fig. 14). An adapted ELISA workflow utilized SARS-CoV-2 patient serum antibodies showed that the RBD produced in BYL was directly comparable to that of RBD produced in HEK293 cells. Interestingly, higher reactivity was observed for COVID-19 patient samples but lower reactivity for samples collected from control subject prior to 2019, and this separation was less signification for S1 antigens. Different C-terminal Strep-II tag and HaloTag configurations did not affect binding, suggesting correct RBD folding independent of fusion protein additions. This experiment suggests the use of RBD produced from BYL CFPS for therapeutic purposes and scaled expression for animal studies is the goal of future research.

### REFERENCES

Buntru, M., Vogel, S., Spiegel, H., & Schillberg, S. (2014). Tobacco BY-2 cell-free lysate: an alternative and highly-productive plant-based in vitro translation system. BMC biotechnology, 14(1), 1-11.
Buntru, M., Vogel, S., Stoff, K., Spiegel, H., & Schillberg, S. (2015). A versatile coupled cell-free transcription-translation system based on tobacco BY-2 cell lysates. Biotechnology and bioengineering, 112(5), 867-878.
Gupta, R., & Brunak, S,. (2002) Prediction of glycosylation across the human proteome and the correlation to protein function. Pac Symp Biocomput. 310-22. PMID: 11928486Henrich, E., Hein, C., Dötsch, V., & Bernhard, F. (2015). Membrane protein production in Escherichia coli cell-free lysates. FEBS letters, 589(15), 1713-1722.
Khambhati, K., Bhattacharjee, G., Gohil, N., Braddick, D., Kulkarni, V., & Singh, V. (2019). Exploring the potential of cell-free protein synthesis for extending the abilities of biological systems. Frontiers in bioengineering and biotechnology, 7, 248.
Sachse, R., Wüstenhagen, D., Samalíková, M., Gerrits, M., Bier, F. F., & Kubick, S. (2013). Synthesis of membrane proteins in eukaryotic cell-free systems. Engineering in Life Sciences, 13(1), 39-48.
Stech, M., Hust, M., Schulze, C., Dübel, S., & Kubick, S. (2014). Cell-free eukaryotic systems for the production, engineering, and modification of scFv antibody fragments. Engineering in life sciences, 14(4), 387-398.
Tian, X., Li, C., Huang, A., Xia, S., Lu, S., Shi, Z., ... & Ying, T. (2020). Potent binding of 2019 novel coronavirus spike protein by a SARS coronavirus-specific human monoclonal antibody. Emerging microbes & infections, 9(1), 382-385.
Yang, L. J., Chen, R. H., Hamdoun, S., Coghi, P., Ng, J. P., Zhang, D. W., ... & Wong, V. K. W. (2021). Corilagin prevents SARS-CoV-2 infection by targeting RBD-ACE2 binding. Phytomedicine, 87, 153591.
Zhou, X., Du, J., Wang, H., Chen, C., Jiao, L., Cheng, X., ... & Gao, Y. (2020). Repositioning liothyronine for cancer immunotherapy by blocking the interaction of immune checkpoint TIGIT/PVR. Cell Communication and Signaling, 18(1), 1-14

## Claims

1. High-throughput screening for at least one ligand of at least one transmembrane protein (TP) of interest that is embedded in a lipid bilayer of at least one endogenous microsome derived from plant-based endoplasmatic reticulum (ER), comprising the steps
- providing at least one endogenous microsome comprising at least one lipid bilayer embedded transmembrane protein (TP) or at least one endogenous microsomal fragment comprising at least one lipid bilayer embedded transmembrane protein (TP),
- providing at least one analyte of interest,
- contacting of the at least one TP with the at least one analyte and
- detection of interaction between the at least one analyte and the at least one TP.

2. The screening according to claim 1, wherein the at least one microsome or microsomal fragment is derived from the genus Nicotiana of the family Solanaceae.

3. The screening according to claim 1 or 2, wherein the at least one TP embedded in a lipid bilayer of the endogenous microsome and/or the endogenous microsomal fragment is captured on a biologically non-active surface.

4. The screening according to any one of the claims 1 to 3, wherein the biologically non-active surface is a surface of a particle comprising micro-particles, nano-particles and magnetic particles, a surface of a device comprising microtiter plates, microfluidic devices, micro arrays, microchips and/or it is any other surface suitable for capturing the at least one TP of interest.

5. The screening according to any one of the claims 1 to 4, wherein the at least one TP comprises at least one tag at the C-terminus and/or N-terminus.

6. The screening according to any one of the claims 1 to 5, wherein the at least one analyte comprises a small molecule, peptide, polypeptide, soluble protein, membrane protein, protein complex or any combination of the aforementioned.

7. The screening according to any one of the claims 1 to 6, wherein it is a multiplex High-throughput screening for at least one ligand of at least one TP of interest providing two or more analytes in two or more reaction zones simultaneously and/or providing an analyte combination of two or more analytes simultaneously in two or more reaction zones.

8. The screening according to any one of the claims 1 to 7, wherein the interaction between the at least one TP and the at least one analyte is detected by means of an immunoassay, a spectroscopic assay, a particle size measurement, a magnetic measurement and/or an optical measurement.

9. The screening according to any one of the claims 1 to 8, wherein the least one endogenous microsome or endogenous microsomal fragment derived from plant-based endoplasmatic reticulum is from a cellular lysate from a plant of the genus Nicotiana of the family Solanaceae.

10. The screening according to any one of the claims 1 to 9, wherein the lysate is from a BY-2 cell line from Nicotiana tabacum.

11. A cell-free production of at least one transmembrane protein (TP) of interest that is embedded in a lipid bilayer of at least one endogenous microsome derived from plant-based endoplasmatic reticulum (ER), comprising the steps
- providing a lysate from a plant comprising at least one endogenous microsome
- providing at least one template encoding for the at least one transmembrane protein (TP),
- incubation of the at least one lysate with the at least one template,
- expression of the at least one transmembrane protein (TP),
- coupled transcription and co-translational translocation of the at least one expressed TP into the at least one microsome and
- obtaining the at least one transmembrane protein (TP) embedded in a lipid bilayer of the at least one endogenous microsome and
- optionally treating the at least one endogenous microsome comprising the at least one TP embedded in a lipid bilayer to obtain microsomal fragments comprising the at least one TP embedded in a lipid bilayer.

12. The method according to claim 11, wherein the at least one microsome or microsomal fragment is derived from the genus Nicotiana of the family Solanaceae.

13. An endogenous microsome or at least one endogenous microsomal fragment derived from plant-based endoplasmatic reticulum (ER) comprising at least one lipid bilayer embedded transmembrane protein (TP) of interest.

14. A biological non active surface comprising at least one captured TP of interest that is embedded in a lipid bilayer of at least one endogenous microsome or of at least one endogenous microsomal fragment derived from plant-based endoplasmatic reticulum (ER), wherein the biologically non active surface is a surface of a consumable.

15. A kit for a high-throughput screening for at least one ligand of at least one transmembrane protein (TP) of interest that is embedded in a lipid bilayer of at least one endogenous microsome derived from plant-based endoplasmatic reticulum (ER), preferably derived from a plant of the genus Nicotiana of the family Solanaceae comprising
- a plant cellular lysate comprising endoplasmatic reticulum (ER) derived endogenous microsomes preferably from a plant of the genus Nicotiana of the family Solanaceae,
- at least one vector encoding for a known TP as positive control
- at least one vector for at least one template encoding for the at least one TP of interest
- optionally further excipients,
- optionally at least one agent for detection of the interaction between the at least one analyte and the at least one TP,
- optionally at least one agent for destruction of the at least one microsome and
- optionally at least one analyte or an analyte panel of two or more analytes.
